# EUROPEAN PATENT APPLICATION

(11) **EP 3 828 541 A1**
(43) Date of publication of application: **02.06.2021**
(21) Application number: 20194399.0
(22) Date of filing: 26.12.2013
(51) Int. Cl.: G01N 33/50, G01N 33/68, G01N 33/566

(54) **BIOMARKERS OF PRETERM BIRTH**

(30) Priority: 28.12.2012 US 201261747150 P; 12.03.2013 US 201313797933
(62) Divisional of application: 13867314.0
(71) Applicant: NX PRENATAL INC., Louisville, KY 40207 (US)
(72) Inventor: EZRIN, Alan, M, Miami, FL 33133 (US); BROHMAN, Brian, D, Prospect, KY 40059 (US)
(74) Representative: Cooley (UK) LLP

(57) **Abstract**

The present disclosure relates to biomarkers of preterm birth, biomarkers of term birth, and methods of use thereof. In particular, the present disclosure provides methods of determining whether a pregnant woman is at an increased risk for premature delivery. The present disclosure further provides methods for decreasing a pregnant woman's risk for premature delivery.

## Description

### CROSS-REFERENCE TO RELATED APPLICATIONS

This application claims the benefit of U.S. Application No. 13/797,933, filed March 12, 2013, and U.S. Provisional Application No. 61/747,150, filed December 28, 2012, which are hereby incorporated by reference in their entirety.

### SUBMISSION OF SEQUENCE LISTING ON ASCII TEXT FILE

The content of the following submission on ASCII text file is incorporated herein by reference in its entirety: a computer readable form (CRF) of the Sequence Listing (file name: 674982000840SEQLIST.txt, date recorded: December 26, 2013, size: 525 KB).

### FIELD

The present disclosure relates to biomarkers of preterm birth, biomarkers of term birth, and methods of use thereof. In particular, the present disclosure provides methods of determining whether a pregnant woman is at an increased risk for premature delivery. The present disclosure further provides methods for decreasing a pregnant woman's risk for premature delivery.

### BACKGROUND

In humans, preterm birth (PTB) is defined as delivery of a pregnancy before 37 weeks of gestation (Honest et al., Health Technology Assessment, 13:1-3, 2009). PTB is associated with significant morbidity and mortality. Premature babies born before 37 weeks, if they survive, have a high incidence of visual and hearing defects, lung disease, developmental delays, and cerebral palsy (Ables and Chauhan, J Family Practice, 54:245-252, 2005).

Preterm deliveries are categorized as elective (about 25% of all premature births) or spontaneous (about 75% of all premature births). Elective preterm deliveries occur as a result of maternal or fetal complications such as hypertension and diabetes (Honest et al., supra, 2009). Spontaneous preterm deliveries occur as a result of preterm labor or preterm premature rupture of membranes (Ables and Chauhan, supra, 2005).

Relatively little progress has been made in determining whether a pregnant woman is at risk for PTB. The lack of tools for identifying risk early in pregnancy coupled with an inability to pinpoint underlying etiology prevents clinicians from proactively detecting and managing the at-risk pregnancy hampering their efforts to improve pregnancy outcomes. In fact, the rate of preterm birth has steadily increased and reflects approximately 15 million of the 187 million births worldwide. In the United States, the preterm birth rate approaches 12.7% resulting in over 500,000 premature infants born each year. Moreover, during the first year of life, between 10 and 20% of premature infants die (March of Dimes, Born Too Soon, 2012; and Society for Maternal Fetal Medicine, High Risk Pregnancy Care, Research, and Education for Over 35 Years, 2010).

A pregnancy ending prematurely is multifactorial in its etiology and remains difficult to predict. For a pregnancy to progress uneventfully, an exquisite balance between numerous physiological systems must be maintained - including the dynamic immunological status of host and fetus at the placental interface. Subtle changes in this balance may be reflected in the identification of a unique set of blood-derived biomarkers found only in pregnancies that end prematurely. The utility and accuracy of novel biomarkers to predict spontaneous preterm birth remains elusive (Conde-Agudelo et al., BJOG, 118:1042-1054, 2011). Further compounding the problem is the complexity of harvesting such markers from biological fluids, the lack of stability of such markers and classic signal to noise issues incumbent to all biomarker studies.

Since the inability to accurately diagnose preterm labor or to determine a pregnant woman's risk of PTB has potentially catastrophic consequences, there is a need in the art for diagnostic and prognostic methods with improved specificity and sensitivity. This need is particularly great considering that timely administration of therapeutic agents may prevent preterm labor or otherwise prolong pregnancy.

### BRIEF SUMMARY

The present disclosure relates to biomarkers of preterm birth, biomarkers of term birth, and methods of use thereof. In particular, the present disclosure provides methods of determining whether a pregnant woman is at an increased risk for premature delivery. The present disclosure further provides methods for decreasing a pregnant woman's risk for premature delivery.

In particular, the present disclosure provides methods for assessing the risk of preterm birth for a pregnant subject, the method comprising: (a) detecting a level of one or more proteins in a sample from the pregnant subject, wherein the one or more proteins are selected from the group consisting of A1BG, AFM, AHSG, ALB, AMBP, APCS, APOA1, APOD, APOH, APOL1, APOM, ATRN, AZGP1, C1QC, C1R, C1S, C3, C4A, C4B, C4BPA, C5, C8A, CD5L, CFB, CFH, CP, CPN1, CPN2, CYP2U1, F12, GSN, HP, HPR, HPX, IGHA1, IGHA2, IGHG1, IGHG4, IGHM, IGHM1, IGHM2, IGHV4-31, IGHVa, IGHVb, IGJ, IGKC, IGKV, IGKV1-9, IGKV3-20, IGKV3D-15, IGLC2, IGLL1, IGLV3-19, IGLV7-43, IGLVa, IGLVb, IGLVc, IGLVd, ITIH1, ITIH2, ITIH3, KNG1, KRT1, KRT10, KRT2, KRT6A, KRT9, KRTAP5-2, LGALS3BP, LPA, PF4, PPBP, PRG2, PZP, PZPs, SAA2-SAA4, SERPINF2, SERPING1, TF, VTN, and VWF; and (b) determining that the pregnant subject is at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is above a threshold level, and/or when the level of one or more of proteins of the term birth group consisting of APCS, APOA1, APOH, C1QC, C1R, C1S, C3, C4A, C4B, C4BPA, C5, CFH, CP, CPN1, CYP2U1, F2, HP, HPX, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is below a threshold level; or determining that the pregnant subject is at not at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is below a threshold level, and/or when the level of one or more of proteins of the term birth group consisting of APCS, APOA1, APOH, C1QC, C1R, C1S, C3, C4A, C4B, C4BPA, C5, CFH, CP, CPN1, CYP2U1, F2, HP, HPX, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is above a threshold level. In some preferred embodiments, the protein is detected by detecting a fragment of the protein (e.g., from four amino acids to full-length minus one amino acid).

The present disclosure provides methods for assessing the risk of preterm birth for a pregnant subject, comprising: (a) preparing a microparticle-enriched fraction from a sample from the pregnant subject; (b) detecting a level of one or more proteins in the fraction, wherein the one or more proteins are selected from the group consisting of A1BG, AFM, AHSG, ALB, AMBP, APCS, APOA1, APOD, APOH, APOL1, APOM, ATRN, AZGP1, C1QC, C1R, C1S, C3, C4A, C4B, C4BPA, C5, C8A, CD5L, CFB, CFH, CP, CPN1, CPN2, CYP2U1, F12, GSN, HP, HPR, HPX, IGHA1, IGHA2, IGHG1, IGHG4, IGHM, IGHM1, IGHM2, IGHV4-31, IGHVa, IGHVb, IGJ, IGKC, IGKV, IGKV1-9, IGKV3-20, IGKV3D-15, IGLC2, IGLL1, IGLV3-19, IGLV7-43, IGLVa, IGLVb, IGLVc, IGLVd, ITIH1, ITIH2, ITIH3, KNG1, KRT1, KRT10, KRT2, KRT6A, KRT9, KRTAP5-2, LGALS3BP, LPA, PF4, PPBP, PRG2, PZP, PZPs, SAA2-SAA4, SERPINF2, SERPING1, TF, VTN, and VWF; and (c) determining that the pregnant subject is at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is above a threshold level, and/or when the level of one or more of proteins of the term birth group consisting of APCS, APOA1, APOH, C1QC, C1R, C1S, C3, C4A, C4B, C4BPA, C5, CFH, CP, CPN1, CYP2U1, F2, HP, HPX, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is below a threshold level; or determining that the pregnant subject is at not at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is below a threshold level, and/or when the level of one or more of proteins of the term birth group consisting of APCS, APOA1, APOH, C1QC, C1R, C1S, C3, C4A, C4B, C4BPA, C5, CFH, CP, CPN1, CYP2U1, F2, HP, HPX, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is above a threshold level. In some preferred embodiments, the protein is detected by detecting a fragment of the protein (e.g., from four amino acids to full-length minus one amino acid).

Additionally, the present disclosure provides methods of decreasing the risk of preterm birth for a pregnant subject, comprising: (a) preparing a microparticle-enriched fraction from a sample from the pregnant subject; (b) detecting a level of one or more proteins in the fraction, wherein the one or more proteins are selected from the group consisting of A1BG, AFM, AHSG, ALB, AMBP, APCS, APOA1, APOD, APOH, APOL1, APOM, ATRN, AZGP1, C1QC, C1R, C1S, C3, C4A, C4B, C4BPA, C5, C8A, CD5L, CFB, CFH, CP, CPN1, CPN2, CYP2U1, F12, GSN, HP, HPR, HPX, IGHA1, IGHA2, IGHG1, IGHG4, IGHM, IGHM1, IGHM2, IGHV4-31, IGHVa, IGHVb, IGJ, IGKC, IGKV, IGKV1-9, IGKV3-20, IGKV3D-15, IGLC2, IGLL1, IGLV3-19, IGLV7-43, IGLVa, IGLVb, IGLVc, IGLVd, ITIH1, ITIH2, ITIH3, KNG1, KRT1, KRT10, KRT2, KRT6A, KRT9, KRTAP5-2, LGALS3BP, LPA, PF4, PPBP, PRG2, PZP, PZPs, SAA2-SAA4, SERPINF2, SERPING1, TF, VTN, and VWF; (c) determining that the pregnant subject is at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is above a threshold level, and/or when the level of one or more of proteins of the term birth group consisting of APCS, APOA1, APOH, C1QC, C1R, C1S, C3, C4A, C4B, C4BPA, C5, CFH, CP, CPN1, CYP2U1, F2, HP, HPX, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is below a threshold level; and (d) administering a therapeutic agent to the subject in an amount effective to decrease the risk of preterm birth. In some preferred embodiments, the protein is detected by detecting a fragment of the protein (e.g., from four amino acids to full-length minus one amino acid).

In some embodiments, the methods of the preceding paragraphs further comprise:
(b2) detecting a level of one or more further proteins in the fraction from the pregnant subject, wherein the one or more further proteins are selected from the group consisting of A2M, APOB, C9, F2, FBLN1, FN1, FN1s, GC, ITIH4, KLKB1, ORM1, ORM2, S100A8, S100A9, SERPINA1, SERPINC1, THBS1, and TTR; and (c2) determining that the pregnant subject is at an increased risk of preterm birth when the level of one or more further proteins of the further preterm birth group consisting of A2M, C9, FBLN1, FN1, FN1s, GC, ITIH4, KLKB1, ORM1, ORM2, S100A8, S100A9, SERPINA1, SERPINC1, and TTR, is above a threshold level, and/or when the level of one or more of further proteins of the further term birth group consisting of APOB, C9, and THBS1 is below a threshold level in the sample. In some embodiments, the detecting step comprises detecting the level of one or more proteins of the preterm group subset consisting of alpha-1B-glycoprotein (A1BG), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), and serotransferrin (TF), and/or
detecting the level of one or more proteins of the term group subset consisting of complement component 1r (C1r), complement component 3 (C3), complement component 4B (C4B), complement factor H (CFH), Ig heavy chain alpha 2 (IGHA2), and Ig kappa variable 1-9 (IGKV1-9). In some embodiments, the detecting step comprises detecting the level of all twelve of alpha-1B-glycoprotein (A1BG), alpha-2-macroglobulin (A2M), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), alpha-1-antitrypsin (SERPINA1), antithrombin-III (SERPINC1), and serotransferrin (TF). In some embodiments, the detecting step comprises detecting the level of all six of complement component 1r (C1r), complement component 3 (C3), complement component 4B (C4B), complement factor H (CFH), Ig heavy chain alpha 2 (IGHA2), and Ig kappa variable 1-9 (IGKV1-9). In some embodiments, the methods comprise a further step after (c) of providing a risk score by enumerating the preterm birth markers above the threshold that were detected and the term birth markers below the threshold that were detected. In some preferred embodiments, the protein is detected by detecting a fragment of the protein (e.g., from four amino acids to full-length minus one amino acid). In some embodiments, the detecting step comprises detecting the level of at least 2, 3, 4, or 5 of the proteins in the fraction, or detecting the level of at least 2, 3, 4, 5, 6, 7, 8, 9, 10, 11, 12, 13, 14, 15, 16, 17, 18, 19 or 20 of the proteins in the fraction. In some embodiments, the detecting step comprises detecting the level of at least 5, at least 10, at least 15, at least 20, at least 25, at least 30, at least 35, at least 40, at least 45, at least 50, at least 55, at least 60, at least 65, at least 70, at least 75, or at least 80 out of the (99) proteins in the fraction. In some embodiments, the pregnant subject is a primigravida woman. In other embodiments, the pregnant subject is a multigravida woman. In some embodiments, the sample is taken from the pregnant subject during the second trimester. In some preferred embodiments, the sample is taken from the pregnant subject within 15 to 17 weeks of gestation. In some embodiments, the sample is blood, saliva, sweat, nasal secretions, tears, urine, amniotic fluid, or cervicovaginal fluid. In some embodiments, the sample is a blood sample, which in preferred embodiment is serum or plasma. In some embodiments, the preparing step does not comprise the use of exoquik or the like. In some embodiments, the preparing step comprises size-exclusion chromatography with an agarose (e.g., SEPHAROSE) solid phase and an aqueous liquid phase. In some embodiments, the aqueous phase is water, while in other embodiments, the aqueous phase is a buffer. In some embodiments the detecting step comprises measuring binding of an antibody specific to each of the one or more proteins. In some embodiments, the detecting step comprises a technique selected from the group consisting of enzyme-linked immunosorbent assay (ELISA), western blot and antibody microarray. In some preferred embodiments, the detecting step comprises liquid chromatography/mass spectrometry (LC/MS). In some embodiments, the therapeutic agent is selected from the group consisting of a hormone, a steroid, intravenous immunoglobulin, and a tumor-necrosis factor-alpha antagonist. In some preferred embodiments the therapeutic agent is progesterone. In some preferred embodiments, the therapeutic agent comprises a cervical cerclage.

### DESCRIPTION OF THE DRAWINGS

**FIG. 1** provides a Western blot analysis of SEC peak 1 material derived from the serum of pregnant women. Six individual patient samples were analyzed. Antigens detected include: FN1, fibronectin; HSP, heat shock protein 70; and CD9, a tetraspanin cell surface protein commonly associated with extracellular vesicles.
**FIG. 2** provides a flow chart of an exemplary method for assessing risk of preterm birth.

### DETAILED DESCRIPTION

Current methods indicate that placental-derived microparticles freely circulate in high titer in maternal blood and reflect the status of the syncytiotrophoblast maternal-fetal interface. Subtle homeostatic changes are reflected in a unique set of dysregulated, microparticle-associated proteins when pregnancy complications occur. As described in more detail in the example section, frozen maternal serum samples (n=48) obtained between 15-17 weeks gestation were analyzed from asymptomatic women subsequently having live births (e.g., excluded women having multiple births, still births, systemic disease, or delivering fetuses with known anomalies). Microparticles were isolated from blood samples using gel filtration, with samples blinded to outcome. Proteins were extracted and analyzed using an open proteomic LC-MS differential analysis between term (>37wks) and preterm cohorts (<34wks). Among 213 proteins identified, a unique pattern was observed in serum microparticles isolated from asymptomatic patients that subsequently delivered preterm. Using an ANOVA assessment and rigorous reproducibility, accuracy, and confidence criteria, 18 proteins were characterized from 99 statistically significant proteins identified across two study phases differentiating the cohorts, with functional analysis implicating inflammatory and cell injury pathways. Thus, the present disclosure provides a library of statistically-valued, microparticle-associated biomarkers that are useful for the early identification of women at risk for preterm birth.

### Definitions

In order to facilitate an understanding of the disclosure, selected terms used in the application will be discussed below.

The term "microparticle" refers to an extracellular microvesicle or lipid raft protein aggregate having a hydrodynamic diameter of from about 50 to about 5000 nm. As such the term microparticle encompasses exosomes (about 50 to about 100 nm), microvesicles (about 100 to about 300 nm), ectosomes (about 50 to about 1000 nm), apoptotic bodies (about 50 to about 5000 nm) and lipid protein aggregates of the same dimensions.

The term "microparticle-associated protein" refers to a protein or fragment thereof (e.g., polypeptide) that is detectable in a microparticle-enriched sample from a mammalian (e.g., human) subject. As such the term "microparticle-associated protein" is not restricted to proteins or fragments thereof that are physically associated with microparticles at the time of detection.

The term "about" as used herein in reference to a value refers to 90 to 110% of that value. For instance a diameter of about 1000 nm is a diameter within the range of 900 nm to 1100 nm.

The phrase "increased risk of preterm birth" as used herein indicates that a pregnant subject has a greater likelihood of having a spontaneous preterm birth (before 38 weeks gestation) when one or more preterm birth markers are detected and/or when one or more term birth markers are not detected. Numerically an increased risk is associated with a hazard ratio of over 1.0, preferably over 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 for preterm birth.

### Protein Biomarkers

The present disclosure relates to tools for assessing and decreasing risk of preterm birth. The methods of the present disclosure include a step of detecting the expression level of a microparticle-associated protein in a biological sample from a pregnant test subject, where the protein is selected from Table I.

**Table I. Microparticle-Associated Proteins Differentially Expressed in Preterm Birth**

| **Protein Name** | **Symbol** | **IPI** | **UniProtKB** |
|---|---|---|---|
| Alpha-2-Macroglobulin | A2M | IPI01010737.1 | P01023 |
| Apolipoprotein B | APOB | IPI00022229.2 | P04114 |
| Apolipoprotein H | APOH | IPI00298828.3 | P02749 |
| Complement C1r | C1R | IPI00956148.2 | Q53HU9 |
| Complement C1s | C1S | IPI00017696.1 | P09871 |
| Complement C3 | C3 | IPI00783987.2 | P01024 |
| Complement Factor H | CFH | IPI00029739.5 | P08603 |
| Immunoglobulin Heavy Chain Mu | IGHM | IPI00418153.1 | Q6N030 |
| Immunoglobulin Heavy Chain Gamma 1 | IGPHG 1 | IPI00384938.1 | Q7Z351 |
| Keratin 1 | KRT1 | IPI00220327.4 | P04264 |
| Keratin 10 | KRT10 | IPI00009865.4 | P13645 |
| Lectin galactoside-binding soluble 3-BP | LGALS3BP | IPI 00023673.1 | Q08380 |
| S100 Calcium-Binding Protein A9 | S100A9 | IPI00027462.1 | P06702 |
| Alpha- 1 -Antitrypsin | SERPINA1 | IPI00553177.1 | P01009 |
| Alpha- 1 B -Glycoprotein | A1BG | IPI00022895.8 | P04217 |
| Albumin | ALB | IPI00745872.2 | P02768 |
| Apolipoprotein L1 | APOL1 | IPI00186903.4 | 014791 |
| Complement C4B (Chido Blood Group) | C4B | IPI00418163.3 | Q6U2E9 |
| Complement C9 | C9 | IPI00022395.1 | P02748 |
| Coagulation Factor XII | F12 | IPI00019581.2 | P00748 |
| Fibronectin 1 short | FN1s | IPI 00922213.2 | B4DTK1 |
| Group-specific component (vitamin D-BP) | GC | IPI00968027.1 | P02774 |
| Immunoglobulin Heavy Chain Alpha 1 | IGHA1 | IPI00449920.1 | P01876 |
| Immunoglobulin Heavy Chain Mu 2 | IGHM2 | IPI00896380.1 | P01871 -1 |
| Immunoglobulin Kappa Constant | IGKC | IPI00979250.1 | P01834 |
| Immunoglobulin Lambda-Like 1 | IGLL1 | IPI00013438.1 | P15814 |
| Inter-alpha-trypsin inhibitor H1 | ITIH1 | IPI00292530.1 | P19827 |
| Inter-alpha-trypsin inhibitor H2 | ITIH2 | IPI00305461.4 | P19823 |
| Inter-alpha trypsin inhibitor H4 | ITIH4 | IPI00944960.1 | B2RMS9 |
| Keratin 6A | KRT6A | IPI00300725.7 | P02538 |
| Pro-platelet basic protein | PPBP | IPI00022445.1 | P02775 |
| Pregnancy zone protein short | PZPLs | IPI00922117.1 | B7Z7M2 |
| Serum amyloid A SAA2-SAA4 | SAA2-SAA4 | IPI00975939.1 | * |
| Afamin | AFM | IPI000 19943.1 | P43652 |
| Alpha-2-HS-glycoprotein | AHSG | IPI00022431.2 | B7Z8Q2 |
| Alpha-1-microglobulin/bikunin precursor | AMBP | IPI00022426.1 | P02760 |
| Amyloid P component, serum | APCS | IPI00022391.1 | P02743 |
| Apolipoprotein A1 | APOA1 | IPI00021841.1 | P02647 |
| Apolipoprotein D | APOD | IPI00924574.1 | C9JF17 |
| Attractin | ATRN | IPI00027235.1 | O75882 |
| Zinc-alpha-2-glycoprotein | AZGP1 | IPI00166729.4 | P25311 |
| Complement C1q C | C1QC | IPI00022394.2 | P02747 |
| Complement C4A (Rodgers Blood Group) | C4A | IPI00843913.3 | B0V2C8 |
| Complement C4 binding protein alpha chain | C4BPA | IPI00021727.1 | P04003 |
| Complement C8 alpha chain | C8A | IPI00011252.1 | P07357 |
| CD5 antigen-like | CD5L | IPI00025204.1 | O43866 |
| Ceruloplasmin | CP | IPI00017601.1 | P00450 |
| Carboxypeptidase N, polypeptide 1 | CPN1 | IPI00010295.1 | P15169 |
| Carboxypeptidase N, polypeptide 2 | CPN2 | IPI00479116.2 | P22792 |
| Coagulation factor II (prothrombin) | F2 | IPI00019568.1 | P00734 |
| Fibulin 1 | FBLN1 | IPI00889740.1 | B1AHL2 |
| Fibronectin 1 | FN1 | IPI00022418.2 | P02751 |
| Haptoglobin | HP | IPI00641737.2 | P00738 |
| Haptoglobin-related protein | HPR | IPI00607707.2 | P00739 |
| Hemopexin | HPX | IPI00022488.1 | P02790 |
| Immunoglobulin Heavy Chain Alpha 2 | IGHA2 | IPI00940245.1 | Q9NPP6 |
| Immunoglobulin Heavy Chain Gamma 4 | IGHG4 | IPI00930442.1 | P01861 |
| Immunoglobulin Heavy Chain Mu 1 | IGHM1 | IPI00479708.6 | P01871 -1 |
| Immunoglobulin Heavy Chain Variable | IGHV | IPI00854743.1 | Q0ZCH6 |
| Immunoglobulin Heavy Chain Variable4-31 | IGHV4-31 | IPI00930124.1 | Q6MZV7 |
| Immunoglobulin J Chain | IGJ | IPI00964840.2 | D6RHJ6 |
| Immunoglobulin Kappa Variable 1-9 | IGKV1-9 | IPI00829751.3 | A2JA19 |
| Immunoglobulin Lambda Constant 2 | IGLC2 | IPI00154742.6 | P0CG05 |
| Immunoglobulin Lambda Variable3-19 | IGLV3-19 | IPI00829640.1 | Q6GMW4 |
| Immunoglobulin Lambda Variable 7-43 | IGLV7-43 | IPI00553092.2 | Q5NV83 |
| Inter-alpha-trypsin inhibitor H3 | ITIH3 | PI:IPI00028413.8 | Q06033 |
| Kallikrein B 1 | KLKB1 | IPI00654888.4 | P03952 |
| Keratin 2 | KRT2 | IPI00021304.1 | P35908 |
| Lipoprotein A | LPA | IPI00029168.1 | P08519 |
| Immunoglobulin Heavy Chain Variable b | IGHVb | IPI00828191.3 | A2NKM7 |
| Alpha-1-acid glycoprotein 1 | ORM1 | IPI00022429.3 | P02763 |
| Alpha-1-acid glycoprotein 2 | ORM2 | IPI00020091.1 | P19652 |
| Platelet Factor 4 | PF4 | IPI00022446.1 | P02776 |
| Pregnancy Zone Protein | PZP | IPI00025426.3 | P20742 |
| Immunoglobulin Kappa Variable | IGKV | IPI00816794.1 | A0N7J6 |
| S100 Calcium Binding Protein A8 | S100A8 | IPI00007047.1 | P05109 |
| Immunoglobulin Kappa Variable3-20 | IGKV3-20 | IPI00916434.1 | A2KBC3 |
| Antithrombin-III | SERPINC1 | IPI00032179.3 | P01008 |
| Alpha-2-antiplasmin | SERPINF2 | IPI00879231.1 | P08697 |
| Plasma protease C1 inhibitor | SERPING1 | IPI00877698.2 | B4E1F0 |
| Transferrin | TF | IPI00022463.2 | P02787 |
| Thrombospondin-1 | THBS1 | IPI00296099.6 | P07996 |
| Transthyretin | TTR | IPI00940791.2 | P02766 |
| Vitronectin | VTN | IPI00298971.1 | P04004 |
| von Willebrand factor | VWF | IPI00023014.3 | P04275 |
| Immunoglobulin Lambda Variable a | IGLVa | IPI00973531.1 | Q7Z2U7 |
| Keratin-associated protein 5-2 | KRTAP5-2 | IPI00555784.1 | Q701N4 |
| Proteoglycan 2 | PRG2 | IPI00847535.1 | P13727 |
| Apolipoprotein M | APOM | IPI00030739.1 | O95445 |
| Complement C5 | C5 | IPI00032291.2 | P01031 |
| Immunoglobulin Lambda Variable b | IGLVb | IPI00890733.1 | B1N7B9 |
| Complement Factor B | CFB | IPI000 19591.2 | Q53F89 |
| Cytochrome P450 2U1 | CYP2U1 | IPI00783946.1 | Q7Z449 |
| Gelsolin | GSN | IPI00026314.1 | P06396 |
| Immunoglobulin Kappa Variable3D-15 | IGKV3D-15 | IPI01026045.1 | Q9UL83 |
| Immunoglobulin Lambda Variable c | IGLVc | IPI00827875.1 | A2NUT2 |
| Immunoglobulin Lambda Variable d | IGLVd | IPI00719373.3 | Q6NS95 |
| Kininogen-1 | KNG1 | IPI00215894.1 | P01042 |
| Keratin 9 | KRT9 | IPI000 19359.4 | P35527 |

### 1. ALPHA-2-MACROGLOBULIN (A2M)

A2M is a secreted protein. The mature protein extends from residues 24-1474, after cleavage of the signal peptide extending from 1-23. A2M is a proteinase inhibitor. It possesses a domain termed the "bait region," which contains specific cleavage sites for all four classes of proteinases. When a proteinase cleaves the bait region, a conformational change is induced in A2M thereby trapping the proteinase. The amino acid sequence of full length A2M is set forth as SEQ ID NO:1 (P01023).

### 2. APOLIPOPROTEIN B-100 (APOB)

APOB is a secreted protein and a major protein constituent of chylomicrons, LDL, and VLDL. The mature protein extends from residues 28-4563, after cleavage of the signal peptide extending from 1-27. APOB contains a vitellogenin protein domain. The amino acid sequence of full length APOB is set forth as SEQ ID NO:2 (P04114).

### 3. APOLIPOPROTEIN H (APOH)

APOH, also known as beta-2-glycoprotein, is a secreted protein. The mature protein extends from residues 20-345, after cleavage of the signal peptide extending from 1-19. APOH contains four CCP/SCR domains. The amino acid sequence of full length APOH is set forth as SEQ ID NO:3 (P02749).

### 4. COMPLEMENT COMPONENT 1, R SUBCOMPONENT (C1R)

C1R is a secreted protein belonging to the peptidase S1 family. The mature protein extends from residues 18-705, after cleavage of the signal peptide extending from 1-17. C1R is further cleaved into two peptides: complement C1r subcomponent light chain (residues 18-462) and heavy chain (residues 464-705). The amino acid sequence of full length C1R is set forth as SEQ ID NO:4 (Q53HU9).

### 5. COMPLEMENT COMPONENT 1, S SUBCOMPONENT (C1S)

C1S is a secreted protein. The mature protein extends from residues 16-688, after cleavage of the signal peptide extending from 1-15. C1S is a serine protease that shows sequence similarity to the peptidase S1 family of proteins. The amino acid sequence of full length C1S is set forth as SEQ ID NO:5 (P09871).

### 6. COMPLEMENT COMPONENT 3 (C3)

C3 is secreted protein. The mature protein extends from 23-1663, after cleavage of the signal peptide extending from 1-22. The mature protein may be further processed into ten fragments. C3 contains both NTR and anaphylatoxin-like domains. The amino acid sequence of full length C3 is set forth as SEQ ID NO:6 (P01024).

### 7. COMPLEMENT FACTOR H (CFH)

CFH is a secreted protein. The mature protein extends from residues 19-1231, after cleavage of the signal peptide extending from 1-18. CFH contains CCP/SCR domains and functions as a cofactor in the inactivation of C3and C5 convertase in the alternate complement pathway. The amino acid sequence of full length CFH is set forth as SEQ ID NO:7 (P08603).

### 8. IMMUNOGLOBULIN HEAVY CHAIN MU (IGHM)

IGHM is an immunoglobulin heavy chain sequence. The amino acid sequence (518 residues) of IGHM is set forth as SEQ ID NO:8 (Q6N030).

### 9. IMMUNOGLOBULIN HEAVY CHAIN GAMMA 1 (IGHG1)

IGHG1 is an immunoglobulin heavy chain sequence. The amino acid sequence (482 residues) of IGHG1 is set forth as SEQ ID NO:9 (Q7Z351).

### 10. KERATIN 1 (KRT1)

KRT1 shows sequence similarity to members of the intermediate filament family of proteins. The amino acid sequence (644 residues) of KRT1 is set forth as SEQ ID NO:10 (P04264).

### 11. KERATIN 10 (KRT10)

KRT10 shows sequence similarity to members of the intermediate filament family of proteins. The amino acid sequence (584 residues) of KRT10 is set forth as SEQ ID NO:11 (P13645).

### 12. LECTIN, GALACTOSIDE-BINDING, SOLUBLE, 3-BINDING PROTEIN (LGALS3BP)

LGALS3BP is secreted protein found ubiquitously in body fluids. The mature protein extends from residues 19-585, after cleavage of the signal peptide extending from 1-18. LGALS3BP contains one of each, BACK, BTB, and SRCR protein domains. The amino acid sequence of full length LGALS3BP is set forth as SEQ ID NO:12 (Q08380).

### 13. S100 CALCIUM-BINDING PROTEIN A9 (S100A9)

S100A9, also known as calgranulin B, extends from residues 1-114. The amino acid sequence of S100A9 is set forth as SEQ ID NO:13 (P06702).

### 14. ALPHA-1-ANTITRYPSIN (SERPINA1)

SERPINA1 is a secreted protein. The mature protein extends from residues 25-418, after cleavage of the signal peptide extending from 1-24. SERPINA1 is an inhibitor of serine proteases, and belongs to the serpin family of proteins. The amino acid sequence of full length SERPINA1 is set forth as SEQ ID NO:14 (P01009).

### 15. ALPHA-1-B-GLYCOPROTEIN (A1BG)

A1BG is a secreted protein. The mature protein extends from residues 22-495, after cleavage of the signal peptide extending from 1-21. A1BG shows sequence similarity to the variable regions of some immunoglobulin supergene family members. The amino acid sequence of full length A1BG is set forth as SEQ ID NO:15 (P04217).

### 16. ALBUMIN (ALB)

ALB is a secreted protein. The mature protein extends from residues 25-609, after cleavage of the signal peptide extending from 1-18 and the propeptide sequence. The amino acid sequence of full length ALB is set forth as SEQ ID NO:16 (P02768).

### 17. APOLIPOPROTEIN L1 (APOL1)

APOL1 is a secreted protein, mainly associated with large high-density lipoprotein particles. The mature protein extends from residues 28-398, after cleavage of the signal peptide extending from 1-27. The amino acid sequence of full length APOL1 is set forth as SEQ ID NO:17 (014791).

### 18. COMPLEMENT COMPONENT 4B (C4B)

C4B is a secreted protein, also known as basic C4 and Chido blood group. The amino acid sequence (1744 residues) of full length C4B is set forth as SEQ ID NO:18 (Q6U2E9).

### 19. COMPLEMENT COMPONENT 9 (C9)

C9 is a secreted protein. The mature protein extends from residues 22-559, after cleavage of the signal peptide extending from 1-21. The mature protein may be further processed into two fragments, C9a, C9b. C9 shows sequence similarity to the complement C6/C7/C8/C9 family of proteins. The amino acid sequence of full length C9 is set forth as SEQ ID NO: 19 (P02748).

### 20. COAGULATION FACTOR XII (F12)

F12 is a secreted protein. The mature protein extends from residues 20-615, after cleavage of the signal peptide extending from 1-19. This mature protein may further be cleaved into four peptides, beta-factor XIIa part 1 and part 2; and coagulation factor XIIa light and heavy chains. F12 shows sequence similarity to the peptidase S1 family of proteins. The amino acid sequence of full length F12 is set forth as SEQ ID NO:20 (P00748).

### 21. FIBRONECTIN 1 short (FN1s)

FN1s is a short isoform of FN1. The amino acid sequence of FN1s is set forth as SEQ ID NO:21 (B4DTK1).

### 22. VITAMIN D-BINDING PROTEIN (GC)

GC is a secreted protein. The mature protein extends from residues17-474, after cleavage of the signal peptide extending from 1-16. GC shows sequence similarity to the ALB/AFP/VDB family of proteins. The amino acid sequence of full length GC is set forth as SEQ ID NO:22 (P02774).

### 23. IMMUNOGLOBULIN HEAVY CHAIN ALPHA 1 (IGHA1)

IGHA1 is an immunoglobulin heavy chain sequence. The amino acid sequence (353 residues) of IGHA1 is set forth as SEQ ID NO:23 (P01876).

### 24. IMMUNOGLOBULIN HEAVY CHAIN MU 2 (IGHM2)

IGHM is an immunoglobulin heavy chain protein having two isoforms, secreted (isoform 1) and single-pass type I membrane (isoform 2). The amino acid sequence of isoform 2, which corresponds to the amino acid sequence (473 residues) of IGHM2 is set forth as SEQ ID NO:24 (P01871-2).

### 25. IMMUNOGLOBULIN KAPPA CONSTANT (IGKC)

IGKC is an immunoglobulin light chain sequence. The amino acid sequence (106 residues) of IGKC is set forth as SEQ ID NO:25 (P01834).

### 26. IMMUNOGLOBULIN LAMBDA-LIKE 1 (IGLL1)

IGLL1 is secreted protein. The mature protein extends from residues 38-213, after cleavage of the signal peptide from 1-17. The amino acid sequence of full length IGLL1 is set forth as SEQ ID NO:26 (P15814).

### 27. INTER-ALPHA-TRYPSIN INHIBITOR H1 (ITIH1)

ITIH1 is a secreted protein. The mature protein extends from residues 35-672, after cleavage of the signal peptide extending from 1-27, and the propeptides extending from 28-34, and 673-911. The amino acid sequence of full length ITIH1 is set forth as SEQ ID NO:27 (P19827).

### 28. INTER-ALPHA-TRYPSIN INHIBITOR H2 (ITIH2)

ITIH2 is a secreted protein. The mature protein extends from residues 55-702, after cleavage of the signal peptide extending from 1-18, and the propeptides extending from 19-54, and 703-946. The amino acid sequence of full length ITIH2 is set forth as SEQ ID NO:28 (P19823).

### 29. INTER-ALPHA-TRYPSIN INHIBITOR H4 (ITIH4)

ITIH4 is a secreted protein. The mature protein extends from residues 29-930, after cleavage of the signal peptide extending from 1-28. This mature protein may further be cleaved into two smaller chains by removal of the propeptide extending from 662-668. The amino acid sequence of full length ITIH4 is set forth as SEQ ID NO:29 (B2RMS9).

### 30. KERATIN 6A (KRT6A)

KRT6A is a cytoskeleton protein. The mature protein extends from residues 2-564, after cleavage of the initiator methionine at position 1. KRT6A shows sequence similarity to the intermediate filament family of proteins. The amino acid sequence of KRT6A is set forth as SEQ ID NO:30 (P02538).

### 31. PRO-PLATELET BASIC PROTEIN (PPBP)

PPBP is a secreted protein. The mature protein extends from residues 35-128, after cleavage of the signal peptide extending from 1-34. PPBP shows sequence similarity to the intercrine alpha family of proteins. The amino acid sequence of full length PPBP is set forth as SEQ ID NO:31 (P02775).

### 32. PREGNANCY ZONE PROTEIN s (PZPs)

PZPs is a short form of PZP. The amino acid sequence of PZPs is set forth as SEQ ID NO:32 (B7Z7M2).

### 33. SERUM AMYLOID A2 PRECURSOR (SAA2-SAA4)

SAA2-SAA4 is a secreted serum amyloid protein precursor. The protein extends from residues 19-208, after cleavage of the signal peptide extending from 1-18. The amino acid sequence of full length SAA2-SAA4 is set forth as SEQ ID NO:33 (GenBank Accession No. NP_001186673).

### 34. AFAMIN (AFM)

AFM is a secreted protein. The mature protein extends from residues 22-599, after cleavage of the signal peptide from 1-21. AFM shows sequence similarity to the ALB/AFP/VDB family of proteins. The amino acid sequence of full length AFM is set forth as SEQ ID NO:34 (P43652).

### 35. ALPHA-2-HS-GLYCOPROTEIN (AHSG)

AHSG is a protein predicted to residue in the extracellular space. AHSG shows sequence similarity to the CY superfamily of proteins. The amino acid sequence (433 residues) of AHSG is set forth as SEQ ID NO:35 (B7Z8Q2).

### 36. ALPHA-1-MICROGLOBULIN/BIKUNIN PRECURSOR (AMBP)

AMBP is a secreted protein. The mature protein extends from residues 20-344, after cleavage of signal peptide from 1-19. The mature protein may be further processed into three peptides, alpha-1-microglobulin, inter-alpha-trypsin inhibitor light chain, and trypstatin. AMBP shows sequence similarity to the calycin superfamily, lipocalin family of proteins. The amino acid sequence of full length AMBP is set forth as SEQ ID NO:36 (P02760).

### 37. AMYLOID P COMPONENT, SERUM (APCS)

APCS is a secreted protein. The mature protein extends from residues 20-223, after cleavage of the signal peptide extending from 1-19. APCS belongs to the pentaxin family of proteins involved in immune response. It can interact with DNA and histones, and may also scavenge nuclear material from damaged circulating cells, as well as function as a calcium dependent lectin. The amino acid sequence of full length APCS is set forth as SEQ ID NO:37 (P02743).

### 38. APOLIPOPROTEIN A1 (APOA1)

APOA1 is a secreted protein. The mature protein extends from residues 25-267, after cleavage of signal peptide from 1-18 and a propeptide. APOA1 shows sequence similarity to the apolipoprotein A1/A4/E family of proteins. The amino acid sequence of full length APOA1 is set forth as SEQ ID NO:38 (P02647).

### 39. APOLIPOPROTEIN D (APOD)

APOD is a secreted protein. The mature protein extends from residues 21-189, after cleavage of the signal peptide extending from 1-20. APOD shows sequence similarity to other members of the alpha-2 microglobulin protein superfamily. The amino acid sequence of APOD is set forth as SEQ ID NO:39 (C9JF17).

### 40. ATTRACTIN (ATRN)

ATRN is a membrane protein (isoform 1); however, isoforms 2 and 3, produced by alternative splicing, are secreted. The mature protein (isoform 1) extends from residues 84-1429, after cleavage of the signal peptide from 1-28, and the propeptide from 29-83. The amino acid sequence of full length ATRN is set forth as SEQ ID NO:40 (O75882).

### 41. ZINC-ALPHA-2-GLYCOPROTEIN 1 (AZGP1)

AZGP1 is a secreted protein. The mature protein extends from residues 21-298, after cleavage of the signal peptide extending from 1-20. AZGPlbelongs to the MHC class I family of proteins, and contains an Ig-like C1-type (immunoglobulin-like) domain. The amino acid sequence of full length AZGP1 is set forth as SEQ ID NO:41 (P25311).

### 42. COMPLEMENT COMPONENT 1, Q SUBCOMPONENT, C CHAIN (C1QC)

C1QC is a secreted protein. The mature protein extends from residues 29-245, after cleavage of the signal peptide extending from 1-28. C1QC contains C1q and collagen-like protein domains. The amino acid sequence of full length C1QC is set forth as SEQ ID NO:42 (P02747).

### 43. COMPLEMENT COMPONENT 4A (C4A)

C4A is a secreted protein, also known as acidic C4 and Rodgers blood group. The amino acid sequence (1744 residues) of full length C4A is set forth as SEQ ID NO:43 (B0V2C8).

**44. COMPLEMENT COMPONENT 4 BINDING PROTEIN ALPHA (C4BPA)** C4BPA is a secreted protein. The mature protein extends from residues 49-597, after cleavage of the signal peptide extending from 1-48. C4BPA contains CCP/SCR domains and controls the classical pathway of complement activation. It also interacts with anticoagulant protein S and serum amyloid P component. The amino acid sequence of full length C4BPA is set forth as SEQ ID NO:44 (P04003).

### 45. COMPLEMENT COMPONENT 8 ALPHA CHAIN (C8A)

C8A is a secreted protein that is part of the membrane attack complex (MAC). The mature protein extends from residues 31-584, after cleavage of the signal peptide extending from 1-20, and the propeptide from 21-30. C8A shows sequence similarity to the complement C6/C7/C8/C9 family of proteins. The amino acid sequence of full length C8A is set forth as SEQ ID NO:45 (P07357).

### 46. CD5 ANTIGEN-LIKE (CD5L)

CD5L is a secreted protein. The mature protein extends from residues 20-347, after cleavage of the signal peptide from 1-19. CD5L contains three SRCR protein domains. The amino acid sequence of full length CD5L is set forth as SEQ ID NO:46 (O43866).

### 47. CERULOPLASMIN (CP)

CP, also known as ferroxidase, is a secreted protein. The mature protein extends from residues 20-1065, after cleavage of the signal peptide extending from 1-19. CP shows sequence similarity to the multicopper oxidase family of proteins. The amino acid sequence of full length CP is set forth as SEQ ID NO:47 (P00450).

### 48. CARBOXYPEPTIDASE N, POLYPEPTIDE 1 (CPN1)

CPN1, also known as anaphylatoxin inactivator, is a secreted protein. The mature protein extends from residues 21-458, after cleavage of the signal peptide extending from 1-20. CPN1 shows sequence similarity to the peptidase M14 family of proteins. The amino acid sequence of full length CPN is set forth as SEQ ID NO:48 (P15169).

### 49. CARBOXYPEPTIDASE N, POLYPEPTIDE 2 (CPN2)

CPN2 is a secreted protein. The mature protein extends from residues 22-545, after cleavage of the signal peptide extending from 1-21. CPN2 contains LLR, LLRCT, and LLRNT protein domains. The amino acid sequence of full length CPN2 is set forth as SEQ ID NO:49 (P22792).

### 50. COAGULATION FACTOR II (F2)

F2, also known as prothrombin, is a secreted protein. The mature protein extends from residues 44-622, after cleavage of the signal peptide extending from 1-24, and the propeptide from 25-43. The mature protein may be further processed into four peptides, activation peptide fragment 1 and 2; and thrombin light and heavy chain. F2 shows sequence similarity to the peptidase S1 family of proteins. The amino acid sequence of full length F2 is set forth as SEQ ID NO:50 (P00734).

### 51. FIBULIN 1 (FBLN1)

FBLN1 is an extracellular matrix protein than contains an EGF-like domain. The mature protein extends from residues 30-703, after cleavage of the signal peptide from 1-29. The amino acid sequence of full length FBLN1 is set forth as SEQ ID NO:51 (B1AHL2).

### 52. FIBRONECTIN (FN1)

FN1 is a secreted protein. The mature protein extends from residues 32-2386, after cleavage of the signal peptide extending from 1-31. The mature protein may be cleaved into four peptides, anastellin, ugl-Y1, ugl-Y2, and ugl-Y3. FN1 contains fibronectin type-I, type-II, and type-III protein domains. There are over a dozen isoforms produced by alternative splicing. The amino acid sequence of FN1 is set forth as SEQ ID NO:52 (P02751).

### 53. HAPTOGLOBIN (HP)

HP is a secreted protein. The mature protein extends from residues 19-406, after cleavage of the signal peptide from 1-18. The mature protein may be further cleaved into two peptides, haptoglobin alpha and haptoglobin beta. HP shows sequence similarity to the peptidase S1 family of proteins. The amino acid sequence of full length HP is set forth as SEQ ID NO:53 (P00738).

### 54. HAPTOGLOBIN-RELATED PROTEIN (HPR)

HPR is a secreted protein. The mature protein extends from residues 20-348, after cleavage of the signal peptide from 1-19. HPR shows sequence similarity to the peptidase S1 family of proteins. The amino acid sequence of full length HPR is set forth as SEQ ID NO:54 (P00739).

### 55. HEMOPEXIN (HPX)

HPX is a secreted protein. The mature protein extends from residues 24-462, after cleavage of the signal peptide extending from 1-23. HPX belongs to a family of proteins involved in transporting heme to the liver for iron recovery. The amino acid sequence of full length HPX is set forth as SEQ ID NO:55 (P02790).

### 56. IMMUNOGLOBULIN HEAVY CHAIN ALPHA 2 (IGHA2)

IGHA2 is an immunoglobulin heavy chain sequence. The amino acid sequence (416 residues) of IGHA2 is set forth as SEQ ID NO: 56 (Q9NPP6).

### 57. IMMUNOGLOBULIN HEAVY CHAIN GAMMA 4 (IGHG4)

IGHG4 is an immunoglobulin heavy chain sequence. The amino acid sequence (327 residues) of IGHG4 is set forth as SEQ ID NO:57 (P01861).

### 58. IMMUNOGLOBULIN HEAVY CHAIN MU 1 (IGHM1)

IGHM is an immunoglobulin heavy chain protein having two isoforms, secreted (isoform 1) and single-pass type I membrane (isoform 2). The amino acid sequence (452 residues) of isoform 1 is set forth as SEQ ID NO:58 (P01871-1).
The amino acid sequence (375 residues) of IGHM1 is set forth as SEQ ID NO:59.

**59. IMMUNGLOBULIN HEAVY CHAIN VARIABLE REGION a (IGHVa)** IGHVa is an immunoglobulin heavy chain variable region sequence that extends from residues 1-131. The amino acid sequence of IGHVa is set forth as SEQ ID NO:60 (QOZCH6).

### 60. IMMUNOGLOBULIN HEAVY CHAIN VARIABLE REGION 4-31 (IGHV4-31)

IGHV4-31 is an immunoglobulin heavy chain sequence. The amino acid sequence (473 residues) of IGHV4-31 is set forth as SEQ ID NO:61 (Q6MZV7).

### 61. IMMUNOGLOBULIN J CHAIN (IGJ)

IGJ is a linker protein that links immunoglobulin monomers (IgM to pentamers, IgA to dimers) and binds these immunoglobulins polymers to the secretory component. The mature protein extends from residues 23-157, after cleavage of the signal peptide extending from 1-22. The amino acid sequence of full length IGJ is set forth as SEQ ID NO:62 (D6RHJ6).

### 62. IMMUNOGLOBULIN KAPPA VARIABLE 1-9 (IGKV1-9)

IGKV1-9 is an immunoglobulin light chain sequence. The amino acid sequence (117 residues) of IGLV is set forth as SEQ ID NO:63.

### 63. IMMUNOGLOBULIN LAMBDA CONSTANT 2 (IGLC2)

IGLC2 is an immunoglobulin light chain sequence. The amino acid sequence of IGLC2 (106 residues) is set forth as SEQ ID NO:64 (P0CG05).

**64. IMMUNOGLOBULIN LAMBDA CHAIN VARIABLE 3-19 (IGLV3-19)** IGLV3-19 is an immunoglobulin light chain sequence. The amino acid sequence (233 residues) of IGLV3-19 is set forth as SEQ ID NO:65 (Q6GMW4).

**65. IMMUNOGLOBULIN LAMBDA CHAIN VARIABLE 7-46 (IGLV7-46)** IGLV7-46 is an immunoglobulin light chain sequence. The amino acid sequence (98 residues) of IGLV7-46 is set forth as SEQ ID NO:66 (Q5NV83).

**66. INTER-ALPHA-TRYPSIN INHIBITOR HEAVY CHAIN H3 (ITIH3)** ITIH3 is a secreted protein. The mature protein extends from residues 35-651, after cleavage of the signal peptide extending from 1-20, and the propeptides from 21-34, and 652-890. The amino acid sequence of full length ITIH3 is set forth as SEQ ID NO:67 (Q06033).

### 67. KALLIKREIN B1 (KLKB1)

KLKB1 is a secreted protein. The mature protein extends from residues 20-638, after cleavage of the signal peptide extending from 1-19. The mature protein may further be cleaved into two peptides, plasma kallikrein heavy chain and light chain. KLKB1 shows sequence similarity to the peptidase S1 family of proteins. The amino acid sequence of full length KLKB1 is set forth as SEQ ID NO:68 (P03952).

### 68. KERATIN (KRT2)

KRT2 is a cytoskeletal protein. KRT2 shows sequence similarity to the intermediate filament family of proteins. The amino acid sequence (639 residues) of KRT2 is set forth as SEQ ID NO:69 (P35908).

### 69. LIPOPROTEIN A (LPA)

LPA is the main constituent of lipoprotein(a). The mature protein extends from residues 20-4548, after cleavage of the signal peptide extending from 1-19. LPA shows sequence similarity to the peptidase S1 family, plasminogen subfamily of proteins. The amino acid sequence of full length LPA is set forth as SEQ ID NO:70 (P08519).

### 70. IMMUNOGLOBULIN HEAVY CHAIN VARIABLE b (IGHVb)

IGHVb (fragment of the NANUC-2 antibody) is an immunoglobulin heavy chain sequence. The amino acid sequence (133 residues) of IGHVb is set forth as SEQ ID NO:71 (A2NKM7).

### 71. OROSOMUCOID-1 (ORM1)

ORM1, also known as alpha-1-acid glycoprotein 1, is a secreted protein. The mature protein extends from residues 19-201, after cleavage of the signal peptide extending from 1-18. ORM1 shows sequence similarity to the calycin superfamily, lipocalin family of proteins. The amino acid sequence of full length ORM1 is set forth as SEQ ID NO:72 (P02763).

### 72. OROSOMUCOID-2 ALPHA-1-ACID GLYCOPROTEIN 2 (ORM2)

ORM2, also known as alpha-1-acid glycoprotein 2, is a secreted protein. The mature protein extends from residues 19-201, after cleavage of the signal peptide extending from 1-18. ORM2 shows sequence similarity to the calycin superfamily, lipocalin family of proteins. The amino acid sequence of full length ORM2 is set forth as SEQ ID NO:73 (P19652).

### 73. PLATELET FACTOR 4 (PF4)

PF4 is a secreted protein that is released during platelet aggregation, and neutralizes the anticoagulant effect of heparin. Mature PF4 protein extends from residues 32-101, after cleavage of the signal peptide extending from 1-31. A shorter form of PF4 extends from 48-101, and is a more potent inhibitor than the longer form. The amino acid sequence of full length PF4 is set forth as SEQ ID NO:74 (P02776).

### 74. PREGNANCY ZONE PROTEIN (PZP)

PZP is a secreted protein. The mature protein extends from residues 26-1482, after cleavage of the signal peptide extending from 1-25. PZP shows sequence similarity to the protease inhibitor 139 family of proteins. The amino acid sequence of full length PZP is set forth as SEQ ID NO:75 (P20742).

### 75. IMMUNGLOBULIN KAPPA CHAIN VARIABLE (IGKV)

IGKV (fragment of the REV25-2 antibody) is an immunoglobulin light chain sequence. The amino acid sequence (134 residues) of IGKV is set forth as SEQ ID NO:76 (AON7J6).

### 76. S100 CALCIUM BINDING PROTEIN A8 (S100A8)

S100A8, also known as calgranulin A, extends from residues 1-93. The amino acid sequence of S100A8 is set forth as SEQ ID NO:77 (P05109).

### 77. IMMUNOGLOBULIN KAPPA VARIABLE 3-20 (IGKV3-20)

IGKV3-20 (scFV) is an immunoglobulin light chain sequence. The amino acid sequence (238 residues) of IGKV3-20 is set forth as SEQ ID NO:78 (A2KBC3).

### 78. ANTITHROMBIN-III (SERPINC1)

SERPINC1 is a secreted protein. The mature protein extends from residues 33-464, after cleavage of the signal peptide extending from 1-32. SERPINC1 is an inhibitor of serine proteases, and belongs to the serpin family of proteins. It is involved in regulating the blood coagulation cascade. The amino acid sequence of full length SERPINC1 is set forth as SEQ ID NO:79 (P01008).

### 79. ALPHA-2-ANTIPLASMIN (SERPINF2)

SERPINF2 is a secreted protein. The mature protein extends from residues 40-491, after cleavage of the signal peptide extending from 1-27 and propeptide from 28-39. The amino acid sequence of full length SERPINF2 is set forth as SEQ ID NO:80 (P08697).

### 80. PLASMA PROTEASE C1 INHIBITOR (SERPING1)

SERPING1 is a protease inhibitor extending from residues 1-505. The amino acid sequence of SERPING1 is set forth as SEQ ID NO:81 (B4E1F0).

### 81. TRANSFERRIN (TF)

TF is a secreted protein. The mature protein extends from residues 20-698, after cleavage of the signal peptide extending from 1-19. The amino acid sequence of full length TF is set forth as SEQ ID NO:82 (P02787).

### 82. THROMBOSPONDIN-1 (THBS1)

THBS1 is a glycoprotein that mediates cell-to-cell and cell-to-matrix interactions. The mature protein extends from residues 19-1170, after cleavage of the signal peptide extending from 1-18. The amino acid sequence of full length THBS1 is set forth as SEQ ID NO:83 (P07996).

### 83. TRANSTHYRETIN (TTR)

TTR is a thyroid hormone-binding protein. The mature protein extends from residues 21-147, after cleavage of the signal peptide extending from 1-20. The amino acid sequence of full length TTR is set forth as SEQ ID NO:84 (P02766).

### 84. VITRONECTIN (VTN)

VTN is a secreted protein. The mature protein extends from 20-478, after cleavage of the signal peptide extending from 1-19. The mature protein may further be cleaved into three peptides, vitronectin V65 subunit, vitronectin V10 subunit, and somatomedin-B. The amino acid sequence of full length VTN is set forth as SEQ ID NO:85 (P04004).

### 85. VON WILLEBRAND FACTOR (VWF)

VWF is a secreted protein. The mature protein extends from 23-2813, after cleavage of the signal peptide extending from 1-22. This mature protein may further be cleaved into two peptides von Willebrand antigen 2, and von Willenbrand factor. The amino acid sequence of full length VWF is set forth as SEQ ID NO:86 (P04275).

### 86. IMMUNOGLOBULIN LAMBDA CHAIN VARIABLE a (IGLVa)

IGLVa is an immunoglobulin light chain sequence extending from residues 1-234. The amino acid sequence of IGLVa is set forth as SEQ ID NO:87 (Q7Z2U7).

### 87. KERATIN-ASSOCIATED PROTEIN 5-2 (KRTAP5-2)

KRTAP5-2 interacts with hair keratins, and extends from residues 1-177. The amino acid sequence of KRTAP5-2 is set forth as SEQ ID NO:88 (Q701N4).

### 88. PROTEOGLYCAN 2 (PRG2)

PRG2 is protein that comes in a proform that is secreted, and a processed form that is found in the matrix of the eosinophil's large specific granule. The mature protein extends from residues 106-222, after cleavage of the signal peptide extending from 1-16, and a propeptide from 17-105. PRG2 contains a C-type lectin domain. The amino acid sequence of full length PRG2 is set forth as SEQ ID NO:89 (P13727).

### 89. APOLIPOPROTEIN M (APOM)

APOM is a secreted protein extending from residues 1-188. APOM binds sphingosine-1-phosphate, myristic acid, palmitic acid and stearic acid, retinol, all-trans-retinoic acid and 9-cis-retinoic acid. APOM shows sequence similarity to the calycin superfamily, lipocalin family of proteins. The amino acid sequence of full length APOM is set forth as SEQ ID NO:90 (O95445).

### 90. COMPLEMENT COMPONENT 5 (C5)

C5 is a secreted protein. A signal peptide extends from 1-18, and a propeptide extends from 674-677. C5 may further be cleaved into four peptides: C5 beta chain, C5 alpha chain, C5a anaphylatoxin, and C5 alpha' chain. The amino acid sequence (1676 residues) of full length C5 is set forth as SEQ ID NO:91 (P01031).

### 91. IMMUNOGLOBULIN LAMBDA CHAIN VARIABLE b

IGLVb (fragment of a cryocrystalglobulin 2 or CC2 antibody) is an immunoglobulin light chain sequence. The amino acid sequence (110 residues) of IGLVb is set forth as SEQ ID NO:92 (B1N7B9).

### 92. COMPLEMENT FACTOR B (CFB)

CFB shows sequence similarity to the peptidase S1 family of proteins. The amino acid sequence (764 residues) of CFB is set forth as SEQ ID NO:93 (Q53F89).

### 93. CYTOCHROME P450 2U1 (CYP2U1)

CYP2U1 is a multi-pass membrane protein extending from residues 1-544. CYP2U1 catalyzes the hydroxylation of arachidonic acid, docosahexaenoic acid and other long chain fatty acids. The amino acid sequence of full length CYP2U1 is set forth as SEQ ID NO:94 (Q7Z449).

### 94. GELSOLIN (GSN)

GSN, also known as actin-depolymerizing factor, is a calcium-regulated, actin-modulating protein that binds to the plus ends of actin monomers or filaments, preventing monomer exchange. GSN exists in two isoforms: isoform 1 is secreted, and isoform 2 is cytoplasmic. The mature isoform 1 protein extends from residues 28-782, after cleavage of the signal peptide extending from 1-27. The mature isoform 2 protein extends from residues 52-782.GSN shows sequence similarity to the villin family of proteins. The amino acid sequence of full length GSN is set forth as SEQ ID NO:95 (P06396).

### 95. IMMUNOGLOBULIN KAPPA VARIABLE 3D-15 (IGKV3D-15)

IGKV3D-15 is an immunoglobulin light chain sequence. The amino acid sequence (108 residues) of IGKV3D-15 is set forth as SEQ ID NO:96 (Q9UL83).

### 96. IMMUNOGLOBULIN LAMBDA CHAIN VARIABLE c (IGLVc)

IGLVc is an immunoglobulin light chain sequence. The amino acid sequence (235 residues) of IGLVc is set forth as SEQ ID NO:97 (A2NUT2).

### 97. IMMUNOGLOBULIN LAMBDA CHAIN VARIABLE d (IGLVd)

IGLVd is an immunoglobulin light chain sequence. The amino acid sequence (234 residues) of IGLVd is set forth as SEQ ID NO:98 (Q6NS95).

### 98. KININOGEN-1 (KNG1)

KNG1 is a secreted protein. The mature protein extends from residues 19-434, after cleavage of the signal peptide extending from 1-18. The mature protein may further be cleaved into six peptides, T-kinin, bradykinin, lysyl-bradykinin, low molecular weight growth-promoting factor, and kininogen-1 heavy and light chains. The amino acid sequence of full length KNG1 is set forth as SEQ ID NO:99 (P01042).

### 99. KERATIN 9 (KRT9)

KRT9 shows sequence similarity to the intermediate filament family of proteins. The amino acid sequence (623 residues) of KRT9 is set forth as SEQ ID NO:100 (P35527).

Microparticle-associated proteins of the present disclosure are not limited to the exemplary amino acid sequences shown above or listed in the sequence database entries of Table I. Rather the protein biomarkers of the present disclosure also include variants of the proteins listed in Table I. "Protein" as used herein refers to a polypeptide or fragment thereof.

"Variants" include proteins having one to several substitution(s), deletion(s), and/or addition(s) of an amino acid in comparison to a reference sequence. Conservative substitution tables providing functionally similar amino acids are well-known in the art. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M).

"Fragments" include polypeptides that are shorter in length than the full length or mature protein of interest. If the length of a protein is x amino acids, a fragment is x-1 amino acids of that protein. The fragment may be shorter than this (e.g., x-2, x-3, x-4, ...), and is preferably 100 amino acids or less (e.g., 90, 80, 70, 60, 50, 40, 30, 20 or 10 amino acids or less). The fragment may be as short as 4 amino acids, but is preferably longer (e.g., 5, 6, 7, 8, 9, 10, 12, 15, 20, 25, 30, 35, 40, 50, 60, 70, 80, 90, or 100 amino acids).

The terms "identical" or percent "identity," in the context of two or more polypeptide sequences, refer to two or more sequences or subsequences that are the same. Two sequences are "substantially identical" if two sequences have a specified percentage of amino acid residues that are the same (e.g., 75%, 80%, 85%, 90%, 95%, 96%, 97%, 98% or 99% identity over a specified region, or, when not specified, over the entire sequence), when compared and aligned for maximum correspondence over a comparison window, or designated region as measured using known sequence comparison algorithms or by manual alignment and visual inspection. Preferably, the identity exists over a region that is at least about 10 amino acids in length, or more preferably over a region that is 20, 50, 100, 200, or more amino acids in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. When comparing two sequences for identity, the sequences do not necessarily have to be contiguous, but any gap would carry with it a penalty that would reduce the overall percent identity. For blastn, the default parameters are Gap opening penalty=5 and Gap extension penalty=2. For blastp, the default parameters are Gap opening penalty=11 and Gap extension penalty=1.

Two examples of algorithms that are suitable for determining percent sequence identity and sequence similarity are the BLAST and BLAST 2.0 algorithms (Altschul et al., Nuc. Acids Res. 25:3389-3402, 1977; and Altschul et al., J. Mol. Biol. 215:403-410, 1990). Software for performing BLAST analyses is publicly available through the National Center for Biotechnology Information. This algorithm involves first identifying high-scoring sequence pairs (HSPs) by identifying short words of length W in the query sequence, which either match or satisfy some positive-valued threshold score T when aligned with a word of the same length in a database sequence. T is referred to as the neighborhood word score threshold. These initial neighborhood word hits act as seeds for initiating searches to find longer HSPs containing them. The word hits are extended in both directions along each sequence for as far as the cumulative alignment score can be increased. Cumulative scores are calculated using, for amino acid sequences, a scoring matrix. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. For amino acid sequences, the BLASTP program uses as defaults a word length of 3, and expectation (E) of 10, and the BLOSUM62 scoring matrix (Henikoff and Henikoff, Proc. Natl. Acad. Sci. USA 89:109-15, 1989) alignments (B) of 50, expectation (E) of 10, M=5, N=-4, and a comparison of both strands.

The BLAST algorithm also performs a statistical analysis of the similarity between two sequences (Karlin and Altschul, Proc. Natl. Acad. Sci. USA 90:5873-5787, 1993). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The present disclosure provides tools for detecting the expression level of a microparticle-associated protein. "Detecting the expression level" includes detecting the presence (e.g., level above a threshold or detectable level) or detecting the absence (e.g., level below a threshold or undetectable level) of a microparticle-associated protein in a sample from a pregnant subject.

During development of the present disclosure numerous microparticle-associated proteins were determined to be elevated in samples from subjects having preterm births (as compared to samples from subjects have term births), and are therefore termed "preterm birth biomarkers." The preterm birth biomarkers identified as described in Example 1 include the following: A1BG, A2M, AFM, AHSG, ALB, AMBP, APOD, APOL1, APOM, ATRN, AZGP1, C8A, C9, CD5L, CFB, CPN2, F12, FBLN1, FN1, FN1s, GC, GSN, HPR, HPX, IGHM1, IGHM2, IGJ, IGLVb, ITIH1, ITIH2, ITIH3, ITIH4, KLKB1, KNG1, KRT1, KRT10, KRT2, KRT6A, LGALS3BP, ORM1, ORM2, PRG2, S100A8, S100A9, SAA2-SAA4, SERPINA1, SERPINC1, SERPINF2, SERPING1, TF, TTR, VTN, and VWF. In some embodiments, the methods of the present invention comprise detecting one or more preterm birth biomarkers selected from the group consisting of: A1BG, AFM, AHSG, ALB, AMBP, APOD, APOL1, APOM, ATRN, AZGP1, C8A, CD5L, CFB, CPN2, F12, GSN, HPR, HPX, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF. In some embodiments, the methods further comprise detecting one or more preterm birth biomarkers selected from the group consisting of A2M, C9, FBLN1, FN1, FN1s, GC, ITIH4, KLKB1, ORM1, ORM2, S100A8, S100A9, SERPINA1, SERPINC1, and TTR. In some preferred embodiments, the methods comprise detecting from four to twelve (e.g.,4, 5, 6, 7, 8, 9, 10, 11 or 12)of the preterm birth biomarkers selected from the group consisting of A1BG, A2M, ALB, APOD, APOL1, AZGP1, HPX, IGHM1, IGHM2, SERPINA1, SERPINC1 and TF.

Additionally during development of the present disclosure numerous microparticle-associated proteins were determined to be reduced in samples from subjects having preterm births (as compared to samples from subjects have term births), and are therefore termed "term birth biomarkers." The term birth biomarkers identified as described in Example 1 include the following: APCS, APOA1, APOB, APOH, C1QC, C1R, C1S, C3, C4A, C4B, C4BPA, C5, C9, CFH, CP, CPN1, CYP2U1, F2, HP, HPX, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, PZPs, and THBS1. In some embodiments, the methods of the present invention comprise detecting one or more term birth biomarkers selected from the group consisting of: APCS, APOA1, APOH, C1QC, C1R, C1S, C3, C4A, C4B, C4BPA, C5, CFH, CP, CPN1, CYP2U1, F2, HP, HPX, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs. In some embodiments, the methods further comprise detecting one or more term birth biomarkers selected from the group consisting of APOB, C9 and THBS1. In some preferred embodiments, the methods comprise detecting from one to six (e.g., 1, 2, 3, 4, 5 or 6) of the term birth biomarkers selected from the group consisting of C1R, C3, C4B, CFH, IGHA2, and IGKV1-9.

### Pregnant Subjects

In some embodiments of the present disclosure, the subject is a pregnant woman. In some embodiments, the pregnant woman is in the first trimester (e.g., weeks 1-12 of gestation), second trimester (e.g., weeks 13-28 of gestation) or third trimester of pregnancy (e.g., weeks 29-37 of gestation). In some embodiments, the pregnant woman is in early pregnancy (e.g., from 8, 9, 10, 11, 12, 13, 14, 15, 16, 17, 18, 19 or 20, but earlier than 21 weeks of gestation; from 20, 19, 18, 17, 16, 15, 14, 13, 12, 11, 10 or 9, but later than 8 weeks of gestation). In some embodiments, the pregnant woman is in mid-pregnancy (e.g., from 21, 22, 23, 24, 25, 26, 27, 28, 29 or 30, but earlier than 31 weeks of gestation; from 30, 29, 28, 27, 26, 25, 24, 23, 22 or 21, but later than 20 weeks of gestation). In some embodiments, the pregnant woman is in late pregnancy (e.g., from 31, 32, 33, 34, 35, 36 or 37, but earlier than 38 weeks of gestation; from 37, 36, 35, 34, 33, 32 or 31, but later than 30 weeks of gestation). The stage of pregnancy can be calculated from the first day of the last normal menstrual period of the pregnant subject.

In some embodiments, the pregnant human subject is primagravida. In other embodiments, the pregnant subject multigravida. In some embodiments, the pregnant subject may have had at least one prior spontaneous preterm birth (e.g., birth prior to week 38 of gestation). In some embodiments, the pregnant human subject is asymnptomatic. In some embodiments, the subject may have a risk factor of PTB such as a history of pre-gestational hypertension, diabetes mellitus, kidney disease, known thrombophilias and/or other significant preexisting medical condition (e.g., short cervical length).

### Samples

A sample for use in the methods of the present disclosure is a biological sample obtained from a pregnant subject prior to 38 weeks of gestation. In preferred embodiments, the sample is collected during a stage of pregnancy described in the preceding section. In some embodiments, the sample is a blood, saliva, tears, sweat, nasal secretions, urine, amniotic fluid or cervicovaginal fluid sample. In some embodiments, the sample is a blood sample, which in preferred embodiments is serum or plasma. In some embodiments, the sample has been stored frozen (e.g., -20°C or -80°C).

### Methods For Assessing Risk of Preterm Birth

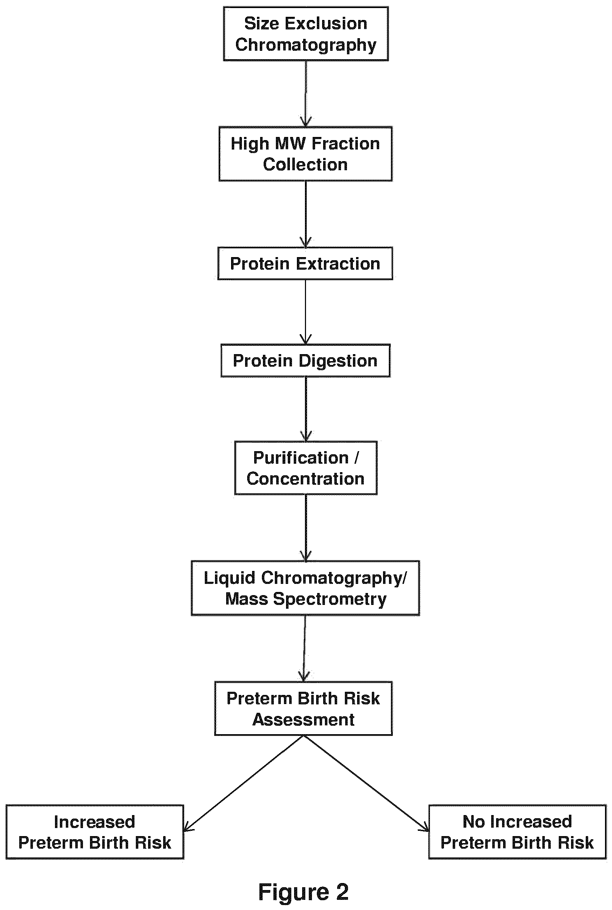

In some embodiments, detecting the expression level (e.g., including detecting the presence) of one or both of preterm birth biomarkers and term birth biomarkers is done using a liquid chromatography / mass spectrometry (LC/MS)- based proteomic analysis such as that depicted in the flow chart of Figure 2. In an exemplary embodiment the method involves subjecting a sample to size exclusion chromatography and collecting the high molecular weight fraction to obtain a microparticle-enriched sample. The microparticle-enriched sample is then extracted before digestion with a proteolytic enzyme such as trypsin to obtain a digested sample comprising a plurality of peptides. The digested sample is then subjected to a peptide purification / concentration step before liquid chromatography and mass spectrometry to obtain a proteomic profile of the sample. In some embodiments, the purification / concentration step comprises reverse phase chromatography (e.g., ZIPTIP pipette tip with 0.2 µL C18 resin, from Millipore Corporation, Billerica, MA). The proteomic profile is analyzed to determine the presence of one or both of preterm birth biomarkers and term birth biomarkers. In some embodiments, preparation of the proteomic profile is done using one or both of the Rosetta ELUCIDATOR data management and analysis system (Ceiba, Boston, MA) and the Mascot search engine (Matrix Systems, Boston MA), which uses mass spectrometry data to identify proteins from primary sequence databases (Pappin et al., Curr Biol, 3:327-332, 1993; Perkins et al., Electrophoresis, 20:35551-2567, 1999; and Koenig et al., J. Proteome Res, 7:3708-3717, 2008).

In some embodiments, detecting the expression level (e.g., including detecting the presence) of one or both of preterm birth biomarkers and term birth biomarkers is done using an antibody-based method. Suitable antibody-based methods include but are not limited to enzyme linked immunosorbent assay (ELISA), Western blot, and antibody microarray.

In some embodiments, the methods include calculating a risk score of preterm birth before gestational week 34 based upon the detection of the expression level of 1 to 18 microparticle-associated proteins of the smallplex consisting of all twelve of alpha-1B-glycoprotein (A1BG), alpha-2-macroglobulin (A2M), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), alpha-1-antitrypsin (SERPINA1), antithrombin-III (SERPINC1), and serotransferrin (TF); and all six of complement component 1r (C1r), complement component 3 (C3), complement component 4B (C4B), complement factor H (CFH), Ig heavy chain alpha 2 (IGHA2), and Ig kappa variable 1-9 (IGKV1-9). The risk score is calculated based on a quintile scale shown in Table II.

**Table II. Quintile Scale for Risk Score Calculation for Smallplex**

| **Preterm Birth Biomarkers above threshold and Term Birth Biomarkers below threshold (# out of 18 total)** | **Score** |
|---|---|
| 0 | 0 (low risk) |
| 1-2 | 1 |
| 3-5 | 2 |
| 6-8 | 3 |
| 9-10 | 4 |
| 11-18 | 5 (high risk) |

### Methods For Reducing Risk of Preterm Birth

Cervical cerclage and progesterone supplementation have been shown to be effective in preventing preterm birth (Committee on Practice Bulletins, Obstetrics & Gynecology, 120:964-973, 2012).

Currently progesterone supplementation for the prevention of recurrent spontaneous preterm birth is offered to: women with a singleton pregnancy and a prior spontaneous preterm birth; and women with no history of spontaneous preterm birth who have an incidentally detected very short cervix (<15 mm). The present disclosure provides tools to identify additional pregnant subjects that may benefit from progesterone supplementation. These subjects include the following: pregnant women who are primigravidas without a history of risk and without an incidentally detected very short cervix; and pregnant women who are multigravidas but who did not previously have a spontaneous preterm birth.

Pregnant subjects determined to be at increased risk for preterm birth are recommended to receive or are administered progesterone until 36 weeks of gestation (e.g., upon identification or between 16 weeks, 0 days and 20 weeks, 6 days gestation until 36 weeks gestation). In some embodiments, progesterone supplementation comprises 250 mg weekly intramuscular injections. In an exemplary embodiment, the weekly progesterone supplementation comprises administration of MAKENA hydroxyprogesterone caproate injection (Ther-Rx Corporation, St. Louis, MO). In other embodiments, progesterone supplementation comprises vaginal progesterone in doses between 50 and 300 mg daily, between 75 and 200 mg daily or between 90 and 110 mg daily.

In another embodiment, in women with a singleton pregnancy determined to be at increased risk for preterm birth and who have had a documented prior spontaneous preterm birth at less than 34 weeks of gestation and short cervical length (less than 25 mm) before 24 weeks of gestation, are recommended to receive or are given a cervical cerclage (also known as tracheloplasty or cervical stitch). In some embodiments, the cervical cerclage is a McDonald cerclage, while in other embodiments it is a Shirodkar cerclage or an abdominal cerclage.

### Kits

In another embodiment, a kit of reagents capable of one or both of preterm birth biomarkers and term birth biomarkers in a sample is provided. Reagents capable of detecting protein biomarkers include but are not limited to antibodies. Antibodies capable of detecting protein biomarkers are also typically directly or indirectly linked to a molecule such as a fluorophore or an enzyme, which can catalyze a detectable reaction to indicate the binding of the reagents to their respective targets.

In some embodiments, the kits further comprise sample processing materials comprising a high molecular gel filtration composition (e.g., agarose such as SEPHAROSE) in a low volume (e.g., 1ml) vertical column for rapid preparation of a microparticle-enriched sample from plasma. For instance, the microparticle-enriched sample can be prepared at the point of care before freezing and shipping to an analytical laboratory for further processing.

In some embodiments, the kits further comprise instructions for assessing risk of preterm birth. As used herein, the term "instructions" refers to directions for using the reagents contained in the kit for detecting the presence (including determining the expression level) of a protein(s) of interest in a sample from a subject. The proteins of interest may comprise one or both of preterm birth biomarkers and term birth biomarkers. In some embodiments, the instructions further comprise the statement of intended use required by the U.S. Food and Drug Administration (FDA) in labeling in vitro diagnostic products. The FDA classifies in vitro diagnostics as medical devices and required that they be approved through the 510(k) procedure. Information required in an application under 510(k) includes: 1) The in vitro diagnostic product name, including the trade or proprietary name, the common or usual name, and the classification name of the device; 2) The intended use of the product; 3) The establishment registration number, if applicable, of the owner or operator submitting the 510(k) submission; the class in which the in vitro diagnostic product was placed under section 513 of the FD&C Act, if known, its appropriate panel, or, if the owner or operator determines that the device has not been classified under such section, a statement of that determination and the basis for the determination that the in vitro diagnostic product is not so classified; 4) Proposed labels, labeling and advertisements sufficient to describe the in vitro diagnostic product, its intended use, and directions for use, including photographs or engineering drawings, where applicable; 5) A statement indicating that the device is similar to and/or different from other in vitro diagnostic products of comparable type in commercial distribution in the U.S., accompanied by data to support the statement; 6) A 510(k) summary of the safety and effectiveness data upon which the substantial equivalence determination is based; or a statement that the 510(k) safety and effectiveness information supporting the FDA finding of substantial equivalence will be made available to any person within 30 days of a written request; 7) A statement that the submitter believes, to the best of their knowledge, that all data and information submitted in the premarket notification are truthful and accurate and that no material fact has been omitted; and 8) Any additional information regarding the in vitro diagnostic product requested that is necessary for the FDA to make a substantial equivalency determination.

### EXAMPLES

Abbreviations: BSA (bovine serum albumin); LC (liquid chromatography); MS (mass spectrometry); P1 (first pregnancy); P2 (second pregnancy); PTB (preterm birth); PT/T (preterm/term ratio); QC (quality control); TIC (total ion current)

### EXAMPLE 1

### Serum Microparticle-Associated Biomarkers Predictive of Risk of Preterm Birth

This example details the proteomic differentiation of maternal serum microparticles obtained from term and preterm patients early in the second trimester. The methods described herein exploit the fact that placental-derived microparticles freely circulate in high titer in maternal blood and reflect the status of the maternal-fetal interface.

### Materials and Methods

*Patient Population.* This study represents a case-controlled analysis of proteomic biomarkers collected from frozen serum samples obtained from early stage pregnant women, from July to November 2009 and released in December 2011, as part of a government initiative under informed consent by Biomnis, France. Non-fasting serum samples were collected for genetic screening and stored in a bio-repository from which a cohort of 168 serum samples were secured and evaluated for study inclusion. Inclusion criteria included normal healthy asymptomatic pregnant women with the absence of a history of pre-gestational hypertension, diabetes mellitus, kidney disease, known thrombophilias or any other significant preexisting chronic medical disease. Medical annotation confirmed gravida conditions (first or second pregnancy), no multiple gestations, live birth, absence of congenital abnormalities and non-in vitro fertilization and non-intracytoplasmic sperm injection pregnancy. Medical annotation from the repository included maternal age, weight, height, and reported tobacco use. Sample data included confirmation of blood draw at weeks 15-17 gestation, infant's birth date and calculation of gestational duration. Outcome status included baby's sex and weight, gestational age (full term birth defined as ≥ 37 weeks gestation), preterm birth (preterm defined as non-iatrogenic spontaneous preterm birth between 23 weeks 0/7 days and 34 weeks 0/7 days gestation).

*Sample Handling.* All samples were frozen -20°C if transit was scheduled for 4 hours or greater from the time of the initial blood draw or refrigerated if transit to the central lab was scheduled to occur less than 4 hours after serum blood draw. After being blinded by identifying the sample number, all serum samples were stored at -80°C (Biomnis, Lyon France). Samples were transported from the repository to the analytical labs (Kannapolis, NC) frozen on dry ice in ∼ 1-2 mL aliquots for the purpose of isolating serum microvesicles and characterizing the proteomic content of serum fractions. Once thawed on the bench at room temperature (∼22°C), microparticles were isolated from 1 mL serum samples by gel filtration methods (Gercel-Taylor et al., Anal Biochem, 428:44-53, 2012). All serum samples were processed and LC/MS analysis was conducted in a double-blinded manner until the end of the study.

*Microparticle Isolation.* Microparticles were isolated from healthy AB serum for quality control and calibration purposes (Sigma Aldrich, St. Louis, MO), and in a blinded manner from term and preterm patient serum samples (n=48). Serum fractions and microparticles were isolated using a 2% such as SEPHAROSE size-exclusion chromatography column (Bio-Rad Econo column 2.5 cm ID x 10 cm with 100 mL of 40% v/v such as SEPHAROSE slurry in ddH20). Samples were processed using a GE AKTA Purifier 10 system (GE Healthcare, Piscataway, NJ) with a Frac 950 fraction collector at room temperature using a flow rate of 2mL/min and a 30 second collection periodicity to obtain 1 mL elution samples.

Serum fractions yielded two partially resolved peaks (peaks 1 and 2) with retention volumes of ∼10 mL and ∼35 mL when monitored by A280. Peak 1 elution samples (< 15 minutes) consisted of high molecular weight fractions of analytical interest that could not permeate the size exclusion column (extracellular vesicles and protein aggregates). The first 7 x 1 mL fractions associated with peak 1 absorbance at A280 were collected, pooled, separated into 7 x 1-mL aliquots for subsequent processing and stored at -80°C. Each pooled fraction was analyzed for total protein and exosomal content quantified and a split aliquot was processed by LC/MS after an ultrafiltration procedure.

The majority of the remaining elution volume and conductivity was associated with the latter peak (∼ 40mL) as might be expected for ions, charged amino acids/peptides and other low molecular weight species (Gercel-Taylor et al., Anal Biochem, 428:44-53, 2012). This parameter was used to demark the beginning of the contaminating second peak, which is devoid of microparticles.

*Microparticle Concentration and Analysis.* A 1 mL aliquot was thawed at room temperature, and subjected to centrifugation at 4,000 x g for approximately 10 min at room temperature using a 1M Da cut off Vivaspin ultrafiltration spin column (Sartorius, Stedim North America Inc. NY) to achieve a 3-fold reduction in sample volume. The spin column was inverted and centrifuged for 5 min at 4,000 x g to collect the retentate harboring the desired microparticles. Total protein concentration in the exosomal preparation was determined using a Thermo Scientific BCA Protein Assay after concentration.

Microvesicular particles (microparticles) were quantified using the Nanoparticle Tracking Analysis (Dragovic et al., Nanomedicine, 7:780-788, 2011) method (NanoSight, LM 10 system, software: NanoSight NTA version 2.2 NanoSight Ltd. Wiltshire, UK). This stochastic single particle detection method is based on light scattering from individual particles using the Stokes-Einstein relationship to measure the average (time-averaged, field of view) number of particles and their predicted hydrodynamic diameter.

The instrument was calibrated and validated with standard beads from 50 - 400 nm in diameter (NanoSight, Wiltshire, UK). One hundred nm beads were run each day to calibrate the system. These notionally 100 nm diameter beads (n >30) yielded experimental diameters of ∼ 97 (±2) nm in aqueous, low viscosity (∼1 cP) solutions at room temperature. All calibrations and patient-derived microvesicles were analyzed in triplicate. Samples were blinded as to cohort of origin and were processed contemporaneously.

*Protein Extraction and Purification.* Proteins were solubilized from the concentrated peak 1 fractions for LC/MS analysis using a modified TRIzol® method (Invitrogen Inc. Carlsbad, CA). Extraction samples were prepared in duplicate and split prior to protein precipitation to form a total of four protein pellets per sample. Initial sample extraction aliquots consisted of 240 µl of cold 50 mM ammonium bicarbonate (stored at 4°C) and 60 µl of concentrated microparticle sample. A 1.0 mL aliquot of TRIzol® Reagent was added and the sample tube was vortexed and incubated at room temperature for 5 minutes. A total of 200 µl of chloroform was added and the sample tube was inverted several times and incubated at room temperature for three minutes. The sample was centrifuged at 12,000 x g for 15 minutes at 4°C on a Microfuge 22R centrifuge. The top aqueous phase was removed and 300 µl of pure ethanol was added to the sample and centrifuged at 2,000 x g at for five minutes at 4°C. The Phenol-ethanol solution was split into two aliquots and 750 µl of isopropyl alcohol was added. The samples were centrifuged at 12,000 x g for 10 minutes at 4°C to pellet the proteins. Each protein pellet was washed in triplicate with 0.3M guanidine HCL followed by a wash with pure ethanol. Protein pellets were suspended in a solution of 50 µl of ammonium bicarbonate (50 mM) and 150 µl of 0.1% RapiGest surfactant (Waters Corporation, Milford, MA)..

The four pellets from sample extraction were combined to provide sufficient material for digestion. A volume of each sample corresponding to 35 µg of protein (based on the protein quantitation results) was used. The sample volumes were made equal by adding 50 mM ammonium bicarbonate (AmBic) to a volume of 110 µL. Disulfide bonds were reduced by adding 200 mM dithiothreitol to each sample and heating the tubes to 80°C for 15 minutes. Preparations were alkylated with 400 mM iodoacetamide (IA) by placing the tubes in the dark for 30 minutes at room temperature. Preparations were digested by adding 1.4 µg trypsin (Promega Corporation, Madison, WI) to each tube and incubating overnight at 37°C. An internal standard, alcohol dehydrogenase digest from yeast (Waters Corporation, Milford, MA), was added to a final concentration of 50 fmol/µg protein. The samples were then concentrated and purified prior to LC/MS analysis using a Millipore ZipTip with 0.2 µL C18 resin (Millipore Corporation, Billerica, MA) eluted with 60µl of a 20% formic acid/80% acetonitrile solution. Samples were dried on a centrifugal vacuum evaporator and reconstituted in 50 µl 0.1% formic acid in water.

*LC*/*MS Quality Control and Chromatography.* Multiple standards were prepared to evaluate method and system performance throughout the study including the use of bovine serum albumin (BSA), which was subjected to reduction/alkylation and digestion in parallel with sample digestion. In addition, a standard *E. coli* digest (Waters) and BSA digest (Michrom) were used to verify instrument performance before and after sample analysis. A 300 fmol injection of the BSA digest was identified by MASCOT with >200 peptide fragments covering greater that 70% of the protein. In addition, a 1 µg injection of an *E. coli* digest resulted in the detection of >250 proteins.

A pooled microparticle QC standard was prepared by mixing 5 µL of each of the 24 microparticle samples and used during analysis of the study sample set. Prior to sample analysis, a minimum of two injections and LC/MS/MS analyses of the pooled microparticle QC standard was performed. Data consistency and system performance were ensured by utilizing an acceptable %RSD of less than 20%. The %RSD of the sample total ion chromatogram was 5.2% and the number of proteins identified had a %RSD of 3.3%

*Analytical Methods.* Analytical sample were analyzed on a Thermo Scientific LTQ Orbitrap XL mass spectrometry system (LTQ Orbitrap XL) coupled to a Waters nanoACQUITY UPLC system (nanoACQUITY UPLC) using standard sample parameters.

Study samples injections were sequenced in a blinded manner and bracketed by a pair of pooled microparticle QC standard injections after every eight sample injections. Data from all study samples were acquired using Data Dependent™ scans (Nth order double play) on the LTQ Orbitrap XL. Database searches were performed in Elucidator (Rosetta Biosoftware) using MASCOT (Matrix Sciences, London, UK).

*Data Analysis.* The pooled microparticle QC and the study samples were evaluated to confirm data quality. Mass spectrometry total ion current (TIC) outputs were assessed for signal quality and changes in signal intensity. Results were also monitored for signal trends, such as a consistent increase or decrease in TIC maximum values, and MASCOT search results were used to monitor the quality of the MS data. No significant outliers were identified during this preliminary evaluation.

Raw MS data files for the pooled microparticle QCs and study samples, collected on the Thermo Orbitrap XL system, were processed in Elucidator. MS data were grouped in Elucidator based on sample groups, identified as group A (term), group B (preterm) and QC. Sample groups were used to assist in data alignment, feature identification, and for QC assessment and group comparisons. Data were processed from retention time 20-180 min.

A label-free differential expression LC/MS/MS method was used to qualitatively compare protein expression levels between two sample groups. The samples were analyzed and the results were processed using the Rosetta Elucidator software package (version 3.3.0.1.SP3_CRE52.21, Rosetta Biosoftware). Data processing in Elucidator was performed in multiple steps that included mass correction and mass signal alignment of detected signals, retention time alignment of detected signals, and feature selection, with features representing mass signals detected within the study set. Mass signals were annotated using the Peptide Tellers function in the Elucidator with the corresponding peptide and protein information based on the database search results using a 1% false discovery rate cut off. Data processing of the Thermo Orbitrap data resulted in detection of ∼200 proteins. QC review of the aligned data indicated the mass signals were correctly aligned in the Elucidator software. For retention time alignment, mathematical adjustments were performed to time align the same detected signals across all samples. QC assessment of this retention time shift indicated that all adjustments had a maximum shift of <1.5 min.

The % CVs across the pooled QC samples and the study samples, grouped into groups A and B, were evaluated to confirm data quality. Data demonstrate that the % CVs for all of the isotope groups monitored across the samples were smaller for the replicate pooled QC injections compared to the A and B sample groups.

Thermo Orbitrap data files were searched using the MASCOT search engine against the IPI (Human, v3.87) database released September 27, 2011 appended with yeast ADH (EMBL-European Bioinformatics Institute, Hinxton, Cambridge, UK). The aligned mass features were annotated with these database search results using the results from the system Peptide Tellers and a predicted error rate of 1%.

MS data were summarized to the feature level, normalized to the mean of the features, and an ANOVA test was performed to compare the expression results between sample groups A and B. Candidate differentially expressed features were determined based on a p<0.05, 0.01 and 0.005. Differentially expressed features were summarized by protein based on the results of the database search.

Data from the studies (n=48 patients representing ∼500,000 peptide fragments), medical annotation and raw intensity and peptide scores were compiled into an automated database for subsequent interrogation. The mean intensity scores were transformed as described by the vendor (Rosetta Inpharmatics Seattle, WA) and blindly analyzed by an independent third party (MF, BIoIT Solutions, Silver Springs, MD).

Significant differences at the peptide and protein levels were characterized in both the primigravida (P1) and second pregnancy (P2) populations. Biological relevance was defined when statistical significance was observed in both P1 and P2 cohorts (p<0.05). In addition, definition of biomarker relevance required that the differential significance defined by the Elucidator system be reconfirmed by blinded third party, that directional ratios in preterm and term cohorts be observed in both P1 and P2 groups, and that the significance was observed in two or more peptide fragments with an average PT/T ratio >1.0. For all determinations the protein teller criteria was set at > 0.9 and false positive protein identification < 1%.

### Results

The demographics of the asymptomatic term and preterm populations (n= 48) presenting between weeks 15 and 17 of gestation are summarized in Table 1-1. No significant differences were noted amongst these groups.

**Table 1-1. Patient Demographics**

| **Parameter** | **Term Birth** | **Preterm Birth** | **p Value** |
|---|---|---|---|
| Sample size | 24 | 24 | |
| Primigravida (P1) | 12 | 12 | NS |
| 2nd pregnancy (P2) | 12 | 12 | NS |
| Maternal age (yrs) | 28.9 +/- 1.75 | 27.75 +/- 3.8 | NS |
| Maternal body mass index | 23.9 +/- 3.57 | 28.4 +/- 4.0 | NS |
| Prior preterm delivery | 0 | 0 | NS |
| Multiparity | 0 | 0 | NS |
| Tobacco use | 1 | 4 | NS |
| Gestational age at delivery (wks) | > 37 | < 34 | |
| Birth weight | 3305 +/- 228 | 2415 +/- 330 | <0.01 |

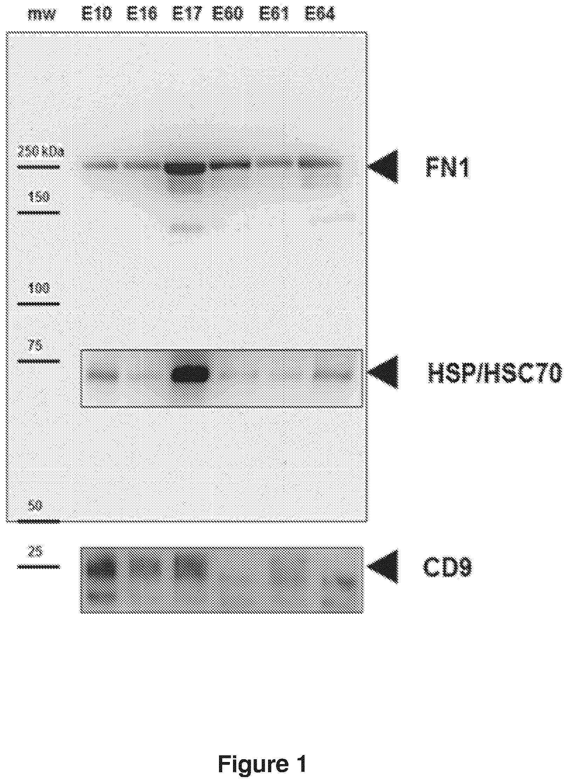

The extracellular vesicles isolated from serum samples eluted by gel filtration methods were characteristic of circulating serum vesicles reported by others (Gercel-Taylor et al., Anal Biochem, 428:44-53, 2012), and expressed canonical proteins indicative of suck particles (FIG. 1)

Serum microparticles isolated from the two groups were not significantly different across any of the phenotypic parameters evaluated at weeks 15-17 gestation including particle density (4.4 - 6.1 x x10¹¹ per ml patient serum), mean particle size (67-109 nm), and protein yield (488 -560 µg/ml sera). The serum vesicles that were isolated from the high molecular (peak 1) fractions, however, differed markedly in protein signature between the preterm and term cohorts.

A total of ∼500,000 peptide fragments were identified in the open proteomic assessment of the 48 patient samples and QC specimens. Study 1 (primigravida P1) identified mass features of 216 proteins with significant differential signal patterns between preterm and term cohorts, which correlated to 85 proteins with putative differentially expressed mass signals based on a cutoff of p< 0.05. Study 2 (second pregnancies P2) identified mass features of 176 proteins with differential signal patterns between preterm and term cohorts using an ANOVA, which correlated to 54 proteins with differentially expressed mass signals based on a cutoff of p< 0.05. Within the proteins observed in the second pregnancies cohort (P2), 32 were observed to be present and statistically significant in the primagravida cohort (P1).

An independent statistical interrogation of the label free Elucidator data were reanalyzed by an independent third party blinded to outcome. These data revealed a total of 99 proteins that were statistically different in expression. From these 99, a total of 18 proteins were selected for validation in future prospective trials. Twelve of the 18 prioritized biomarkers are highly associated with preterm outcome (Table 1-2), while six of the 18 are associated with term outcome (Table 1-3).

**Table 1-2. Microparticle Preterm Birth Biomarkers**

| **Symbol** | **Protein Name** | **p Value** |
|---|---|---|
| A1BG | Alpha-1B-glycoprotein | 0.01 |
| A2M | Alpha-2-macroglobulin | 0.005 |
| ALB | Albumin | 0.01 |
| APOD | Apolipoprotein D | 0.05 |
| APOL1 | Apoliprotein L1 | 0.01 |
| AZGP1 | Zinc-alpha-2-glycoprotein | 0.05 |
| HPX | Hemopexin | 0.05 |
| IGHM1 | IgM Heavy Chain 1 (secreted) | 0.05 |
| IGHM2 | IgM Heavy Chain 2 (membrane-bound) | 0.01 |
| SERPINA1 | Alpha-1 -anti-trypsin | 0.005 |
| SERPINC1 | Anti-thrombin | 0.05 |
| TF | Transferrin | 0.05 |

**Table 1-3. Microparticle Term Birth Biomarkers**

| **Symbol** | **Protein Name** | **p Value** |
|---|---|---|
| C1R | Complement C1r | 0.005 |
| C3 | Complement C3 | 0.005 |
| C4B | Complement C4B | 0.01 |
| CFH | Complement Factor H | 0.005 |
| IGHA2 | IgA Heavy Chain | 0.05 |
| IGKV1-9 | Ig Kappa Variable 1-9 | 0.05 |

The multiplex analysis allowed for a differential characterization of proteins and patients into hierarchical relationships of cohort relevance. Table 1-4 summarizes the accuracy and false positive rates derived from the data. The multiplexed characterization of accuracy based upon cluster analysis revealed a high level of precision in accurately defining patients at risk as early as week 15 for unexpected preterm delivery prior to week 34. The associated probability for the described events has a sensitivity range from 85.4-97.9 % (41-47 accurate detection in 48 cases) depending upon the probability of error level set for the window of discrimination. The false positive rates ranged from 3-7 out of 48 samples evaluated, again depending upon the probability of error level chosen.

**Table 1-4. Predictive Value of Term and Spontaneous Preterm Birth Markers (N=48)**

| Probability Factors | Accurate Out of Expected | | Accurate Out of Actual | |
|---|---|---|---|---|
| | Term | SPTB | Term | SPTB |
| 0.05 | 20/24 | 21/24 | 20/23 | 21/25 |
| 0.01 | 22/24 | 22/24 | 22/24 | 22/24 |
| 0.005 | 23/24 | 23/24 | 24/25 | 23/23 |

Tables 1-5 and 1-6 summarize the functional analysis of the markers identified in both the term and SPTB cohorts. Using Ingenuity Systems Pathway Analysis Software (IPA, Redwood, CA, USA), a detailed comparison of the molecular pathway analysis of the microvesicle proteins expressed in each group revealed that the biomarkers in the SPTB population were associated with five major pathways and different markedly from the pattern in the term population when evaluated at weeks 15-17 of gestation. The focus molecules detected in the SPTB microparticles largely reflect immune, inflammatory and injury pathways. In contrast, the focus molecules in the term microparticles detected early in the second trimester were associated with cell-to-cell signaling.

**Table 1-5. Ingenuity Pathway Analysis in SPTB Microparticles**

| **ID** | **Molecules in Network** | **Score** | **Focus Molecule** | **Top Functions** |
|---|---|---|---|---|
| 1 | A1BG, AHSG, AMBP, APOD, C9, C4B (includes others), Complement component 1, Cytokeratin, elastase, F12, FBLN1, GC, HDL, HPR, HPX, lti, ITIH1, ITIH2, ITIH3, ITIH4, Kallikrein, KLKB1, KRT1, KRT2, KRT10, KRT6A, NFkB (complex), SERPINC1, SERPINF2, SERPING1, Tcf 1/3/4, trypsin, TTR, VTN | 67 | 26 | Antigen Presentation, Humoral Immune Response, Inflammatory Response |
| 2 | A2M, AFM, ALB, Ap1, collagen, Collagen type IV, Collagen(s), Creb, ERK1/2, Fibrinogen, FN1, GPIIB-IIIA, Growth hormone, Iga, Ige, IgG1, IgG, IGHM*, IGJ, IGLL1/IGLL5, IL1, Immunoglobulin, Insulin, Integrin alpha V beta 3, Laminin, LDH, LDL, LGALS3BP, LRP, ORM1/ORM2, S100A8, SERPINA1, TF, TGF beta, vWF | 29 | 13 | Organismal Injury and Abnormalities, Respiratory Disease, Tissue Morphology |
| 3 | Akt, Alpha catenin, AZGP1, CCL15, CD3, CD27, CHCHD2, chemokine, CPN2, HTRA1, IGLL1/IGLL5, IL17R, Integrin, Interferon alpha, Jnk, Mapk, MMP11, Mmp, Neurotropin, P38 MAPK, PAX3, PDESA, PI3K (complex), PIK3C2B, Pkc(s), PRSS1/PRSS3, Ptk, S100, S100A8, S100A9, S100B, SLC3A2, TFEC, TNFRSF4, Vegf | 9 | 5 | Cell-to-Cell Signaling and Interaction, Inflammatory Response, Cardiovascular Disease |
| 4 | APP, C2, C5, C6, C7, C8, C9, C4B (includes others), C5-C6-C7-C8-C9, C8A, C8B, C8G, CD59 (includes EG: 25407), Cd59a, CD5L, CFB, chondroitin sulfate B, CHRNA7, chymotrypsin, Complement compound 1, CPB2, CPN1, GPR77, HABP2, hemoglobin, IL1B, membrane attack, methylamine, PPP1R15A, PRDX2, S100B, Serine Protease, SMPD2, SYVN1, TNFAIP2 | 8 | 5 | Antigen Presentation, Humoral Immune Response, Inflammatory Response |
| 5 | ADCYAP1 (includes EG: 11516), ASIP, ATRN, COPSS | 2 | 1 | Cell-to-Cell Signaling and Interaction, Cellular Assembly and Organization, Cellular Growth and Proliferation |

**Table 1-6. Ingenuity Pathway Analysis in Term Microparticles**

| **ID** | **Molecules in Network** | **Score** | **Focus Molecule** | **Top Functions** |
|---|---|---|---|---|
| 1 | APCS, APOA1, APOB, APOH, C3, C1q, C1QC, C1R, C1S, C4B (includes others), C4BP, C4BPA, CFH, Collagen(s), Complement component 1, CP, Fibrin, Fibrinogen, HDL, IgG1, IgG, IgG2a, IGHG4, IGHM, IGKC, Igm, IL1, Immunoglobulin, LDL, LDL-cholesterol, LPA, NFkB (complex), PF4, Pro-inflammatory Cytokine, SYK/ZAP | 44 | 17 | Cell- To-Cell Signaling and Interaction, Tissue Development, Antigen Presentation |
| 2 | Akt, BAG4, C3, Cd55/Daf2, chemokine, CR1L, CXCL16, CXCR7, ERK, ERK1/2, F2, GALNT2, GEM, HABP2, IGHM, IL12 (family), immune, jnk, Mac1, MGAT3, MS4A1, P38 MAPK, PDGF BB, PI3K (complex), Pkc(s), PLA2G6, plasminogen activator, PPBP, PZP, Rac, SERPINF2, SLAMF1, TGFBI (includes EG: 21803), THBS1, Vegf | 12 | 6 | Inflammatory Response, Cellular Movement, Hematological System Development and Function |
| 3 | CD40, CDK2API, ELANE, FCAR, IGHA1, IGHA2, IL6, IL8, ITGAM (includes EG: 16409), ITGB2, MBL2, Mucin, NFKBIA, PI3K (complex), RELA, TCF3, TGF beta, TLR4, TNF | 4 | 2 | Cell- To-Cell Signaling and Interaction, Antigen Presentation, Inflammatory Response |

The use of extracellular vesicles circulating in high titer in blood as windows to understand disease processes has recently gained considerable attention in the field of molecular biology (Olver and Vidal, Subcell Biochem, 43:99-131, 2007; Simpson et al., Proteomics, 8:4083-99, 2008; Thery, F1000 Biol Rep, 3:15, 2011; and Pant et al., Biochem Pharmacol, 83:1484-94, 2012). The present study demonstrates the clinical utility of using circulating extracellular vesicles as enrichment factors to identify protein biomarkers indicative of risk for preterm birth. A key advantage in utilizing a microparticle-enriched fraction is that it contains protease-resistant microvesicles of endocytic origin (e.g., exosomes), which are thought to capture relevant biomarkers of disease processes (Simpson et al., supra 2008). In addition to containing nucleic acid and protein markers reflective of the tissue of origin, these shed particles have been reported to play an immunomodulatory role in normal pregnancy (Taylor et al., J Immunol, 176:1534-1542, 2006; Sabapatha et al., Am J Reprod Immunol, 56:345-55, 2006; and Atay et al., Am J Reprod Immunol, 65:65-77, 2011).

The present disclosure is an in-depth, open proteomic evaluation of microvesicular biomarkers harvested from maternal serum confirming the use of multiplexed proteins as early warning signals of preterm birth. It is of interest to note that the methods used in this example do not employ expensive protein extraction and affinity purification schemes such as affinity columns for the removal of the high abundant proteins (Tang et al., J Proteome Res, 10:4005-4017, 2011). Thus these methods allow for the rapid and cost effective isolation microparticles from a biological sample from a patient. In addition to the intrinsic benefit of studying microparticle proteomics, the fractionation process allows for detection of the sub proteome proteins outside the typical high abundant species. It is of interest to note that six (e.g., A1BG, APOD, APOL1, AZGP1, HPX and SERPINC1) of the prioritized twelve biomarkers associated with preterm birth are outside of the classical 20 species associated with high abundance proteins that define 97% of the proteome. That is in an exemplary embodiment, highly abundant plasma proteins such as albumin, IgG, IgA, IgM, IgD, transferrin, fibrinogen, alpha2-macroglobulin, alpha1-antitrypsin, haptoglobin, alpha1-acid glycoprotein, ceruloplasmin, APOA1, APOA2, APOB, C1q, C3, C4, plasminogen and prealbumin are not depleted from the blood sample.

The protein biomarkers characterized as discrete entities in this study have been previously described in circulating exosomes (www.exocarta.com) but have never been defined as unique or multiplexed markers associated with preterm birth. Several protein biomarkers identified in the present study have been previously defined as potential serum biomarkers in preterm birth. Previous interest in using α1 antitrypsin (Stella et al., Am J Obstet Gynecol, 201:387, 2009), anti-thrombin (Esplin et al., Am J Obstet Gynecol, 204:391, 2011) and alpha-2-macroglobulin in recurrent pregnancy loss (Saunders et al., Am J Reprod Immunol, 68:438-49, 2012) as circulating biomarker has been reported. The present study represents the first demonstration of differential expression of these and other proteins as markers for preterm risk as early as weeks 15 to 17 of gestation in an asymptomatic population.

The present disclosure confirms the utility of a rapid isolation from sera (30 minutes versus classical 30 hour extractions) of circulating microparticles. Unique protein biomarkers were evaluation using standard LC/MS procedures amenable to a routine CLIA facility. The intended use of such an assay is to be utilized as a non-invasive test to identify pregnant women at risk for premature birth as early as 15 weeks gestation. This would represent a first in human test utilizing a standard blood sample taken between weeks 15 and 17 of gestation. The 1 ml maternal serum sample is sent to a central lab for LC/MS processing and risk results provided in 2-4 days with a targeted risk profile for the later onset of preterm birth. Importantly, this would permit the health care provider and the pregnant subject to take steps to reduce her risk of preterm birth.

The invention can be further defined with reference to the following embodiments or clauses:
1. A method for assessing risk of preterm birth for a pregnant subject, the method comprising:
   (a) preparing a microparticle-enriched fraction from a sample from the pregnant subject;
   (b) detecting a level of one or more proteins in the fraction, wherein the one or more proteins are selected from the group consisting of A1BG, AFM, AHSG, ALB, AMBP, APCS, APOA1, APOD, APOH, APOL1, APOM, ATRN, AZGP1, C1QC, C1R, C1S, C3, C4A, C4B, C4BPA, C5, C8A, CD5L, CFB, CFH, CP, CPN1, CPN2, CYP2U1, F12, GSN, HP, HPR, HPX, IGHA1, IGHA2, IGHG1, IGHG4, IGHM, IGHM1, IGHM2, IGHV4-31, IGHVa, IGHVb, IGJ, IGKC, IGKV, IGKV1-9, IGKV3-20, IGKV3D-15, IGLC2, IGLL1, IGLV3-19, IGLV7-43, IGLVa, IGLVb, IGLVc, IGLVd, ITIH1, ITIH2, ITIH3, KNG1, KRT1, KRT10, KRT2, KRT6A, KRT9, KRTAP5-2, LGALS3BP, LPA, PF4, PPBP, PRG2, PZP, PZPs, SAA2-SAA4, SERPINF2, SERPING1, TF, VTN, and VWF; and
   (c) determining that the pregnant subject is at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is above a threshold level, and/or when the level of one or more of proteins of the term birth group consisting of APCS, APOA1, APOH, C1QC, C1R, C1S, C3, C4A, C4B, C4BPA, C5, CFH, CP, CPN1, CYP2U1, F2, HP, HPX, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is below a threshold level; or
      determining that the pregnant subject is at not at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is below a threshold level, and/or when the level of one or more of proteins of the term birth group consisting of APCS, APOA1, APOH, C1QC, C1R, C1S, C3, C4A, C4B, C4BPA, C5, CFH, CP, CPN1, CYP2U1, F2, HP, HPX, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is above a threshold level.
2. The method of clause 1, further comprising:
   (b2) detecting a level of one or more further proteins in the fraction from the pregnant subject, wherein the one or more further proteins are selected from the group consisting of A2M, APOB, C9, F2, FBLN1, FN1, FN1s, GC, ITIH4, KLKB1, ORM1, ORM2, S100A8, S100A9, SERPINA1, SERPINC1, THBS1, and TTR; and
   (c2) determining that the pregnant subject is at an increased risk of preterm birth when the level of one or more further proteins of the further preterm birth group consisting of A2M, C9, FBLN1, FN1, FN1s, GC, ITIH4, KLKB1, ORM1, ORM2, S100A8, S100A9, SERPINA1, SERPINC1, and TTR, is above a threshold level, and/or when the level of one or more of further proteins of the further term birth group consisting of APOB, C9, and THBS1 is below a threshold level in the sample.
3. The method of clause 1, wherein the detecting step comprises detecting the level of one or more proteins of the preterm group subset consisting of alpha-1B-glycoprotein (A1BG), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), and serotransferrin (TF), and/or detecting the level of one or more proteins of the term group subset consisting of complement component 1r (C1r), complement component 3 (C3), complement component 4B (C4B), complement factor H (CFH), Ig heavy chain alpha 2 (IGHA2), and Ig kappa variable 1-9 (IGKV1-9).
4. The method of clause 1, wherein the detecting step comprises detecting the level of all twelve of alpha-1B-glycoprotein (A1BG), alpha-2-macroglobulin (A2M), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), alpha-1-antitrypsin (SERPINA1), antithrombin-III (SERPINC1), and serotransferrin (TF).
5. The method of clause 1, wherein the detecting step comprises detecting the level of all six of complement component 1r (C1r), complement component 3 (C3), complement component 4B (C4B), complement factor H (CFH), Ig heavy chain alpha 2 (IGHA2), and Ig kappa variable1-9 (IGKV1-9).
6. The method of clause 1, comprising a further step after (c) of providing a risk score by enumerating the preterm birth markers above the threshold that were detected and the term birth markers below the threshold that were detected.
7. The method of clause 1, wherein the detecting step comprises detecting the level of at least 2, 3, 4, or 5 of the proteins in the fraction.
8. The method of clause 1, wherein the pregnant subject is a primigravida woman.
9. The method of clause 1, wherein the pregnant subject is a multigravida woman.
10. The method of clause 1, wherein the sample is taken from the pregnant subject during the second trimester.
11. The method of clause 10, wherein the sample is taken from the pregnant subject within 15 to 17 weeks of gestation.
12. The method of clause 1, wherein the sample is a blood sample.
13. The method of clause 12, wherein the blood sample is serum or plasma.
14. The method of clause 1, wherein the preparing step does not comprise the use of exoquik.
15. The method of clause 1, wherein the preparing step comprises size-exclusion chromatography with an agarose solid phase and an aqueous liquid phase.
16. The method of clause 15, wherein the aqueous liquid phase is water.
17. The method of clause 1, wherein the detecting step comprises measuring binding of an antibody specific to each of the one or more proteins.
18. The method of clause 17, wherein the detecting step comprises a technique selected from the group consisting of enzyme-linked immunosorbent assay (ELISA), western blot and antibody microarray.
19. The method of clause 1, wherein the detecting step comprises liquid chromatography/mass spectrometry (LC/MS).
20. A method of decreasing risk of preterm birth for a pregnant subject, the method comprising:
   (a) preparing a microparticle-enriched fraction from a sample from the pregnant subject;
   (b) detecting a level of one or more proteins in the fraction, wherein the one or more proteins are selected from the group consisting of A1BG, AFM, AHSG, ALB, AMBP, APCS, APOA1, APOD, APOH, APOL1, APOM, ATRN, AZGP1, C1QC, C1R, C1S, C3, C4A, C4B, C4BPA, C5, C8A, CD5L, CFB, CFH, CP, CPN1, CPN2, CYP2U1, F12, GSN, HP, HPR, HPX, IGHA1, IGHA2, IGHG1, IGHG4, IGHM, IGHM1, IGHM2, IGHV4-31, IGHVa, IGHVb, IGJ, IGKC, IGKV, IGKV1-9, IGKV3-20, IGKV3D-15, IGLC2, IGLL1, IGLV3-19, IGLV7-43, IGLVa, IGLVb, IGLVc, IGLVd, ITIH1, ITIH2, ITIH3, KNG1, KRT1, KRT10, KRT2, KRT6A, KRT9, KRTAP5-2, LGALS3BP, LPA, PF4, PPBP, PRG2, PZP, PZPs, SAA2-SAA4, SERPINF2, SERPING1, TF, VTN, and VWF;
   (c) determining that the pregnant subject is at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is above a threshold level, and/or when the level of one or more of proteins of the term birth group consisting of APCS, APOA1, APOH, C1QC, C1R, C1S, C3, C4A, C4B, C4BPA, C5, CFH, CP, CPN1, CYP2U1, F2, HP, HPX, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is below a threshold level; and
   (d) administering a therapeutic agent to the subject in an amount effective to decrease the risk of preterm birth.
21. The method of clause 20, wherein the therapeutic agent is selected from the group consisting of a hormone, a steroid, intravenous immunoglobulin, and a tumor-necrosis factor-alpha antagonist.
22. The method of clause 20, wherein the therapeutic agent is progesterone.

## Claims

1. A method for assessing risk of preterm birth for a pregnant subject, the method comprising:
(a) preparing a microparticle-enriched fraction from a sample from the pregnant subj ect;
(b) detecting a level of one or more proteins in the fraction, wherein the one or more proteins are selected from the group consisting of SERPING1, A1BG, AFM, AHSG, ALB, AMBP, APCS, APOA1, APOD, APOH, APOL1, APOM, ATRN, AZGP1, C1QC, C1R, C1S, C3, C4A, C4B, C4BPA, C5, C8A, CD5L, CFB, CFH, CP, CPN1, CPN2, CYP2U1, F12, GSN, HP, HPR, HPX, IGHA1, IGHA2, IGHG1, IGHG4, IGHM, IGHM1, IGHM2, IGHV4-31, IGHVa, IGHVb, IGJ, IGKC, IGKV, IGKV1-9, IGKV3-20, IGKV3D-15, IGLC2, IGLL1, IGLV3-19, IGLV7-43, IGLVa, IGLVb, IGLVc, IGLVd, ITIH1, ITIH2, ITIH3, KNG1, KRT1, KRT10, KRT2, KRT6A, KRT9, KRTAP5-2, LGALS3BP, LPA, PF4, PPBP, PRG2, PZP, PZPs, SAA2-SAA4, SERPINF2, TF, VTN, and VWF, wherein the protein or at least one of the proteins is SERPING1; and
(c) determining that the pregnant subject is at an increased risk of preterm birth when the level of one or more proteins consisting of SERPING1, A1BG, AFM, AHSG, ALB, AMBP, APOD, APOL1, APOM, ATRN, AZGP1, C8A, CD5L, CFB, CPN2, F12, GSN, HPR, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, TF, VTN, and VWF is above a threshold level, optionally, by providing a risk score by enumerating the preterm birth markers above the threshold that were detected.

2. The method of claim 1, further comprising:
(b2) detecting a level of one or more further proteins in the fraction from the pregnant subject, wherein the one or more further proteins are selected from the group consisting of A2M, APOB, C9, F2, FBLN1, FN1, FN1s, GC, ITIH4, KLKB1, ORM1, ORM2, S100A8, S100A9, SERPINA1, SERPINC1, THBS1, and TTR; and
(c2) determining that the pregnant subject is at an increased risk of preterm birth when the level of one or more further proteins consisting of A2M, C9, FBLN1, FN1, FN1s, GC, ITIH4, KLKB1, ORM1, ORM2, S100A8, S100A9, SERPINA1, SERPINC1, and TTR, is above a threshold level, and/or when the level of one or more of further proteins consisting of APOB, C9, and THBS1 is below a threshold level in the sample.

3. The method of claim 1, wherein the detecting step comprises detecting the level of one or more proteins consisting of alpha-1B-glycoprotein (A1BG), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), and serotransferrin (TF), and/or detecting the level of one or more proteins consisting of complement component 1r (C1r), complement component 3 (C3), complement component 4B (C4B), complement factor H (CFH), Ig heavy chain alpha 2 (IGHA2), and Ig kappa variable1-9 (IGKV1-9).

4. The method of claim 1, wherein the detecting step comprises detecting the level of one or more proteins consisting of alpha-1B-glycoprotein (A1BG), alpha-2-macroglobulin (A2M), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), alpha-1-antitrypsin (SERPINA1), antithrombin-III (SERPINC1), and serotransferrin (TF).

5. The method of any one of the preceding claims, wherein the detecting step comprises detecting the level of one or more proteins consisting of complement component 1r (C1r), complement component 3 (C3), complement component 4B (C4B), complement factor H (CFH), Ig heavy chain alpha 2 (IGHA2), and Ig kappa variable1-9 (IGKV1-9).

6. The method of any one of the preceding claims, wherein the detecting step comprises detecting the level of at least 2, 3, 4, 5, 6, 7, 8, 9, 10, 11, 12, 13, 14, 15, 16, 17, 18, 19 or 20 of the proteins in the fraction.

7. The method of any one of the preceding claims, wherein the pregnant subject is a primigravida woman or a multigravida woman.

8. The method of any one of the preceding claims, wherein the sample:
(a) is taken from the pregnant subject during the first trimester, second trimester or third trimester of pregnancy;
(b) is taken from the pregnant subject within 8 to 20 weeks of gestation; or
(c) is a blood sample, optionally, wherein the blood sample is serum or plasma.

9. The method of any one of the preceding claims, wherein the preparing step comprises size-exclusion chromatography with an agarose solid phase and an aqueous liquid phase, optionally, wherein the aqueous liquid phase is water.

10. The method of any one of the preceding claims, wherein the detecting step comprises measuring binding of an antibody specific to each of the one or more proteins, optionally, wherein the detecting step comprises a technique selected from the group consisting of enzyme-linked immunosorbent assay (ELISA), western blot and antibody microarray.

11. The method of any one of the preceding claims, wherein the detecting step comprises liquid chromatography/mass spectrometry (LC/MS).

12. A method of decreasing risk of preterm birth for a pregnant subject, the method comprising:
(a) preparing a microparticle-enriched fraction from a sample from the pregnant subj ect;
(b) detecting a level of one or more proteins in the fraction, wherein the one or more proteins are selected from the group consisting of SERPING1, A1BG, AFM, AHSG, ALB, AMBP, APCS, APOA1, APOD, APOH, APOL1, APOM, ATRN, AZGP1, C1QC, C1R, C1S, C3, C4A, C4B, C4BPA, C5, C8A, CD5L, CFB, CFH, CP, CPN1, CPN2, CYP2U1, F12, GSN, HP, HPR, HPX, IGHA1, IGHA2, IGHG1, IGHG4, IGHM, IGHM1, IGHM2, IGHV4-31, IGHVa, IGHVb, IGJ, IGKC, IGKV, IGKV1-9, IGKV3-20, IGKV3D-15, IGLC2, IGLL1, IGLV3-19, IGLV7-43, IGLVa, IGLVb, IGLVc, IGLVd, ITIH1, ITIH2, ITIH3, KNG1, KRT1, KRT10, KRT2, KRT6A, KRT9, KRTAP5-2, LGALS3BP, LPA, PF4, PPBP, PRG2, PZP, PZPs, SAA2-SAA4, SERPINF2, TF, VTN, and VWF, wherein the protein or at least one of the proteins is SERPING1; and
(c) determining that the pregnant subject is at an increased risk of preterm birth when the level of one or more proteins consisting of SERPING1, A1BG, AFM, AHSG, ALB, AMBP, APOD, APOL1, APOM, ATRN, AZGP1, C8A, CD5L, CFB, CPN2, F12, GSN, HPR, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, TF, VTN, and VWF is above a threshold level, optionally, by providing a risk score by enumerating the preterm birth markers above the threshold that were detected; and
(d) administering a therapy to the subject in an amount effective to decrease the risk of preterm birth.

13. The method of claim 12, wherein the therapy;
(a) is a therapeutic agent selected from the group consisting of a hormone, a steroid, intravenous immunoglobulin, and a tumor-necrosis factor-alpha antagonist, optionally, wherein the therapeutic agent is progesterone; or
(b) is cervical cerclage.

14. The method of any one of the preceding claims, wherein the protein is detected by detecting a fragment of the protein from four amino acids to full-length minus one amino acid.
